(19) Europäisches Patentamt
European Patent Office
Office européen des brevets

(11) EP 4 534 742 A2

(12) # EUROPEAN PATENT APPLICATION

published in accordance with Art. 153(4) EPC

(43) Date of publication:
**09.04.2025 Bulletin 2025/15**

(21) Application number: **23815236.7**

(22) Date of filing: **31.05.2023**

(51) International Patent Classification (IPC):
***C40B 40/00*** (2006.01) ***C40B 30/04*** (2006.01)
***C40B 30/06*** (2006.01) ***C40B 70/00*** (2006.01)

(52) Cooperative Patent Classification (CPC):
**C40B 30/04; C40B 30/06; C40B 40/00; C40B 70/00**

(86) International application number:
**PCT/CN2023/097328**

(87) International publication number:
**WO 2023/232058 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.06.2022 CN 202210613328**

(71) Applicant: **Wholesome Future Limited**
**Grand Cayman KY1-1103 (KY)**

(72) Inventor: **ZHAO, Haifeng**
**Beijing 100036 (CN)**

(74) Representative: **Laine IP Oy**
**Porkkalankatu 24**
**00180 Helsinki (FI)**

(54) **POLYPEPTIDE-ENCODED LIBRARY AND SCREENING METHOD USING SAME**

(57) Provided is a polypeptide-encoded nucleic acid or small molecule compound library. The library comprises polypeptide barcode tags and nucleic acid molecules or small molecule compounds corresponding to the polypeptide barcode tags. Further provided is a polypeptide-encoded nucleic acid or small molecule compound library, the library comprising a plurality of beads, and each bead comprising a polypeptide barcode tag connected to the bead and a nucleic acid molecule or a small molecule compound corresponding to the polypeptide barcode tag. Further provided is a method for screening a target nucleic acid or small molecule via the polypeptide-encoded nucleic acid or small molecule compound library, wherein a screened nucleic acid molecule or small molecule compound is identified by decoding a polypeptide barcode tag associated therewith.

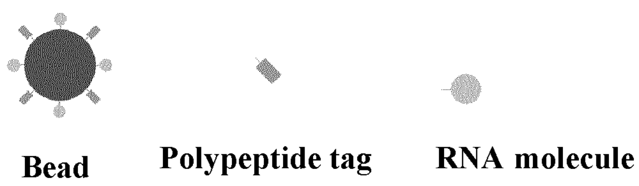


FIG. 1

EP 4 534 742 A2

Processed by Luminess, 75001 PARIS (FR)

## Description

### Technical Field

The present invention relates to the technical field of biology and medical devices. Specifically, the present invention provides a polypeptide encoded library, especially a polypeptide encoded nucleic acid library and a small molecule compound library, as well as a method and microfluidic device for screening a polypeptide encoded library.

### Technical Background

With the development of biotechnology, various small molecule compounds and biomacromolecules are increasingly widely used to treat and prevent diseases. This field requires high-throughput screening of a large number of small molecule compounds and biomacromolecules (including nucleic acid molecules such as mRNA molecules) candidates.

### Summary of the Invention

The present invention provides a polypeptide encoded nucleic acid library or polypeptide encoded small molecule compound library, the library comprising a plurality of nucleic acid molecules or small molecule compounds, wherein each of the nucleic acid molecules or small molecule compounds has a corresponding polypeptide barcode tag. The nucleic acid library or small molecule compound library provided by the present invention can be used to screen candidate molecules that are beneficial for disease diagnosis or treatment, and is particularly suitable for high-throughput and rapid screening of libraries containing a large number of nucleic acid molecules or small molecule compounds.

In one aspect of the present invention, the library is a small molecule compound library. Small molecules generally refer to molecules with a molecular weight of less than about 1000 Daltons. Small molecules can be organic or inorganic, isolated (for example, from a compound library or natural source) or obtained by derivatization of known compounds.

In one aspect of the present invention, the library is a nucleic acid library. Nucleic acids include DNA and RNA. In another aspect of the present invention, the library is an mRNA or RNAi library.

A tag refers to information that uniquely identifies a nucleic acid molecule or small molecule compound on a same microparticle, thereby serving as a unique identifier for the specific nucleic acid molecule or small molecule compound. Therefore, the tag encodes a specific nucleic acid molecule or small molecule compound and serves as a molecular "barcode". In the present invention, the tag is a polypeptide, and the information is encoded in the amino acid residue sequence.

In one aspect of the present invention, the polypeptide barcode tag is formed by sequentially connecting n amino acid residue building blocks. Where n is an integer of 3-10, preferably an integer of 4-8, for example, n is 4 or 5 or 6. Wherein the nucleic acid molecule or small molecule compound is formed by sequentially connecting m nucleic acid units or small molecule compound building blocks, wherein m is the same as the number n of amino acid residue building blocks in the polypeptide barcode tag on the corresponding bead. In another aspect of the present invention, the nucleic acid units or small molecule compound building blocks and the amino acid residue building blocks in each nucleic acid molecule or small molecule compound in the library are sequentially and alternately assembled with the amino acid residue building blocks, so that the polypeptide barcode tag composed of the multiple amino acid residue building blocks is unique and forms a corresponding relationship with each nucleic acid molecule or small molecule compound: according to the sequence information of the polypeptide barcode tag, the composition information of the corresponding nucleic acid molecule can be obtained, including the nucleic acid sequence, nucleoside type, etc. determined by the above multiple nucleic acid units, or the group composition and structure of the small molecule compound.

In one aspect of the present invention, a polypeptide encoded nucleic acid library or polypeptide encoded small molecule compound library is provided, the library includes a plurality of beads, each bead includes a polypeptide barcode tag connected to the bead and a nucleic acid molecule or small molecule compound corresponding to the polypeptide barcode tag. **In** one embodiment of the present invention, the nucleic acid molecule or small molecule compound is releasably connected to the bead.

**In** one aspect of the present invention, the polypeptide barcode tag is formed by sequentially connecting n amino acid residue building blocks. Wherein n is an integer of 3-10, preferably an integer of 4-8, for example, n is 4 or 5 or 6. Wherein the nucleic acid molecule or small molecule compound is formed by sequentially connecting m nucleic acid units or small molecule compound building blocks, wherein m is the same as the number n of amino acid residue building blocks in the polypeptide barcode tag on the corresponding bead. Wherein, the multiple amino acid residue building blocks are sequentially connected in series and connected to the bead surface, wherein all the multiple amino acid residue building blocks together constitute the polypeptide barcode tag. And wherein the multiple nucleic acid units or small molecule

compound building units are sequentially connected in series and connected to the bead surface, and all the multiple nucleic acid units together constitute the nucleic acid molecule. **In** another aspect of the present invention, each nucleic acid unit or small molecule compound building unit and each amino acid residue building unit are sequentially and alternately assembled onto the bead. The nucleic acid units or small molecule compound building blocks and the amino acid residue building blocks are assembled in sequence and alternately, so that the polypeptide barcode label formed on each bead by the multiple amino acid residue building blocks is unique, and at the same time forms a corresponding relationship with the nucleic acid molecules or small molecule compounds composed of the multiple nucleic acid units on the same bead: according to the sequence information of the polypeptide barcode label, the composition information of the corresponding nucleic acid molecule can be obtained, including the nucleic acid sequence, nucleoside type, etc. determined by the above multiple nucleic acid units, or the group composition and structure of the small molecule compound.

**In** the present invention, the synthesis and production of beads usually adopts the "split and pool" method. Generally, the "split and pool" method is used to simultaneously synthesize bead-bound nucleic acids or small molecule compounds and bead-bound polypeptide barcodes. For example, the method involves the following steps: (a) dividing the beads into different containers; (b) adding a different structural unit (nucleic acid unit or compound building unit) to each container. For example, in the case of using three containers, the first nucleic acid unit member or compound building unit A is added to the first container and reacted, the first nucleic acid unit member or compound building unit B is added to the second container; and the first nucleic acid unit member or compound building unit C is added to the third container, wherein the nucleic acid unit is covalently bound to the attachment site on any bead in the container; (c) all beads are pooled in one container; (d) the beads are redistributed to the three containers, (e) another different nucleic acid unit or compound building unit is added to each container, wherein the second nucleic acid unit member or compound building unit A is added to the first container, the second nucleic acid unit member or compound building unit B is added to the second container, and the second nucleic acid unit member or compound building unit C is added to the third container, wherein the second nucleic acid unit or compound building unit member is bound to the first nucleic acid unit or compound building unit member that has been previously attached. At the same time, before or after each nucleic acid unit member or compound building unit binding step, a step of coupling amino acid residue building units of a polypeptide barcode tag is included. A feature of the bead-bound library prepared by the split and merge method is that each bead will have only one nucleic acid molecule or compound molecule attached thereto and a corresponding unique polypeptide barcode tag.

In the present invention, the number of different nucleic acid molecules contained in the library is determined by the number of groups of nucleic acid units and the number of member nucleic acids in each group of nucleic acid units, and its number increases exponentially with the number of synthesis steps (determining the number of groups of nucleic acid units) and building blocks (i.e., the number of members in each group of nucleic acid units). This strategy enables the number of nucleic acid molecules in the library to reach the scale of hundreds of millions or even hundreds of billions. For example, when 5 groups of mRNA units (e.g., the 5' cap, 5' untranslated region, i.e., 5'UTR, mRNA coding sequence, 3' untranslated region, i.e., 3'UTR, and polyadenylates tail of mRNA in order) are used for synthesis, and each group includes 10 members, a library containing $10^5$ mRNA molecules will be generated. For another example, when 10 groups of mRNA units (e.g., 10 fragments of mRNA coding sequences) are used for synthesis, and each group includes 5 members, a library containing $5^{10}$ mRNA molecules will be generated. Each bead in the library has multiple copies of one of the mRNA molecule libraries and has a unique polypeptide barcode tag corresponding to the mRNA bound thereto. According to the sequence information of the polypeptide barcode tag of each bead, the composition information of the corresponding nucleic acid molecule can be obtained, including the nucleic acid sequence, nucleoside type, etc. determined by the above-mentioned multiple nucleic acid units.

In one aspect of the present invention, the polypeptide barcode tags of different nucleic acid molecules or small molecule compounds contained in the library are decoded by sequencing. In one aspect of the present invention, the sequencing is performed by mass spectrometry, and the information of the polypeptide barcode is read out by mass spectrometry of the sample. In one aspect of the present invention, the units (single amino acids or a combination of a specified number of amino acid residues) of the polypeptides used to form the barcode tags in the library each have different (and known) characteristics that can be identified by mass spectrometry, including molecular weight, etc., or including fragmentation rules, elution time and/or isotope distribution. The corresponding mass spectrometry features can be mapped to the nucleic acid molecules or small molecule compounds on the corresponding beads. The polypeptide barcode tag is formed by multiple units, such as individual amino acids or a specified number of amino acid residue combinations. Each unit has a distinguishable mass spectrometry feature. The mass spectrometry feature thus identifies each unit of the polypeptide barcode tag itself, its position in the barcode sequence, etc. The polypeptide can be sequenced using various known mass spectrometry analysis methods and instruments known in the art. The mass spectrometry analysis method generally includes transferring the ionized peptide molecules to the vacuum system of the mass spectrometer for mass spectrometry scanning, which records the mass spectrum of the peptide entering the instrument during the scan. Quantification is performed based on the peaks present in this mass spectrometry (MS) scan.

In one aspect of the present invention, each amino acid residue unit in the polypeptide barcode tag comprises 1 or

more (for example, 2-8, preferably 2-3) amino acid residues. In another aspect of the present invention, the amino acid residue unit comprises 1 or 2 amino acid residues.

In the present invention, the amino acid residues in the polypeptide barcode tag can be various natural or synthetic amino acid residues and their modifications or variants. Differently labeled (e.g., lightly labeled or heavily labeled) amino acid residues can be used to increase the discrimination of polypeptide barcodes based on different masses. In one aspect of the present invention, the amino acid residues include modified amino acid residues, such as lightly labeled or heavily labeled amino acid residues. Preferably, the amino acid residues are amino acid residues containing heavy isotopes. The optional heavy isotopes are selected from deuterium (D), $^{13}C$ and $^{15}N$. Amino acid residues containing heavy isotopes are, for example, L-arginine-$^{13}C_6$, L-arginine-$^{13}C_6{}^{15}N_4$, L-arginine-$^{13}C_6{}^{15}N_4D_7$, L-arginine-$^{15}N_4D_7$, L-arginine-$^{15}N_4$, L-lysine-$^{13}C_6{}^{15}N_2$, L-lysine-$^{15}N_2$, L-lysine-$^{13}C_6$, L-lysine-$^{13}C_6{}^{15}N_2D_9$, L-lysine-$^{15}N_2D_9$, L-lysine-$D_4$, L-methionine-$^{13}CD_3$, L-tyrosine-$^{13}C_9$, L-tyrosine-$^{15}N$ and L-tyrosine-$^{13}C_9{}^{15}N$.

In one aspect of the present invention, the beads are beads made of metal and/or polymer materials, such as polystyrene beads. In the present invention, the beads may also be hydrogel particles, such as gel particles such as polyacrylamide, agarose, and alginate. The surface or interior of the beads has functional groups that can produce covalent or ionic or hydrogen bonds, and polypeptides and nucleic acid molecules can be fixed on the beads directly or through connecting groups.

In one aspect of the present invention, the size of the beads is between 1$\mu$m and 100$\mu$m, preferably between 1$\mu$m and 20$\mu$m.

As mentioned above, in one aspect of the present invention, the library is a small molecule compound library. The small molecule compounds in the library are composed of small molecule compound building blocks. The small molecule compound building block can be any useful chemical structural unit, including inorganic or organic groups, such as optionally substituted alkyl or cycloalkyl groups (e.g., optionally substituted linear or branched C1-6 alkyl groups or optionally substituted C1-6 aminoalkyl groups), optionally substituted aromatic groups (e.g., optionally substituted phenyl or benzyl), optionally substituted heterocyclic groups (e.g., optionally substituted quinolyl, isoquinolyl, indolyl, pyridyl, piperidinyl or pyrrolidinyl), or optionally substituted carbocyclic groups (e.g., optionally substituted cyclopropyl, cyclohexyl or cyclohexenyl).

As mentioned above, in one aspect of the present invention, the library is a ribonucleic acid, i.e., RNA molecule library. In one aspect of the present invention, the RNA molecule is mRNA or RNAi. Each mRNA or RNAi molecule in the library has a variety of different structural or chemical modification designs.

The modifications carried by the mRNA and/or RNAi molecules in the library can make them have better structural or chemical characteristics than natural or synthetic mRNA and/or RNAi molecules, and obtain better biological activity and effects. For example, it can be used to optimize nucleic acid-based therapeutics while retaining structural and functional integrity, overcome expression thresholds, increase expression rate, half-life and/or protein concentration, optimize protein localization, and avoid harmful biological responses, such as immune responses and/or characteristics of degradation pathways. Modified mRNA and/or RNAi molecules may improve stability and/or clearance in tissues, receptor uptake and/or kinetics, cellular entry of the composition, engagement with the translation machinery, half-life, translation efficiency, immune evasion, protein production capacity, secretion efficiency (when applicable), circulation accessibility, protein half-life and/or cell state, function and/or activity tuning. In addition, the modified mRNA and/or RNAi has lower immunogenicity.

Modifications of mRNA and/or RNAi include any useful modifications, such as modifications to saccharides, nucleobases, or internucleoside bonds (e.g., to connecting phosphate/phosphodiester bonds/phosphodiester backbones). For example, the major groove of an mRNA or RNAi or the major groove surface of a nucleobase may contain one or more modifications. One or more atoms of the pyrimidine nucleobase (such as on the large groove surface) can be replaced or substituted by an optionally substituted amino group, an optionally substituted thiol, an optionally substituted alkyl group (such as methyl or ethyl) or a halogen (such as chlorine or fluorine). In certain embodiments, each sugar and internucleoside bond has a modification (such as one or more modifications). The modification according to the present invention can be to modify ribonucleic acid (RNA) to deoxyribonucleic acid (DNA), such as, 2'OH of the ribofuranosyl ring is replaced by 2'H, threose nucleic acid (TNA), glycol nucleic acid (GNA), peptide nucleic acid (PNA), locked nucleic acid (LNA) or its hybrid).

In another aspect of the present invention, the nucleic acid molecule is RNAi. RNAi includes but is not limited to siRNA, shRNA and miRNA. The term "interfering RNA" or "RNAi" or "interfering RNA sequence" refers to a single-stranded RNA (e.g., mature miRNA) or double-stranded RNA (i.e., duplex RNA, such as siRNA, aiRNA or pre-miRNA) that can reduce or inhibit the expression of a target gene or sequence when the interfering RNA and the target gene or sequence are in the same cell, and the interfering RNA therefore refers to a single-stranded RNA complementary to the target mRNA sequence or a double-stranded RNA formed by two complementary chains or a single self-complementary chain. Interfering RNA can have substantially or completely the same identity as the target gene or sequence, or can include a mismatch region (i.e., a mismatch motif). The sequence of interfering RNA can correspond to the full-length target gene or its subsequence. Interfering RNA includes "small-interfering RNA" or "siRNA", such as an interfering RNA

with a length of about 15-60, 15-50 or 5-40 (duplex) nucleotides. The siRNA duplex may comprise a 3' overhang and a 5' phosphate end of about 1 to about 4 nucleotides or about 2 to about 3 nucleotides. Examples of siRNA include, but are not limited to, a double-stranded polynucleotide molecule assembled from two separate chain molecules, one of which is a sense strand and the other is a complementary antisense strand; a double-stranded polynucleotide molecule assembled from a single chain molecule, wherein the sense and antisense regions are connected by a nucleic acid-based or non-nucleic acid-based linker; a double-stranded polynucleotide molecule with a hairpin secondary structure, wherein the hairpin secondary structure has self-complementary sense and antisense regions; and a circular single-stranded polynucleotide molecule having two or more loop structures and a stem with self-complementary sense and antisense regions, wherein the circular polynucleotide can be processed in vivo or in vitro to produce an active double-stranded siRNA molecule. Small hairpin RNA or short hairpin RNA (shRNA) is an RNA sequence that makes a tight hairpin bend, which can be used to silence gene expression via RNA interference. The shRNA hairpin structure is cut into siRNA by the cell machinery, which then binds to the RNA-induced silencing complex (RISC). The complex binds to and cleaves mRNA that matches the siRNA bound thereto. Suitable lengths of RNAi include, but are not limited to, about 5 to about 200 nucleotides, or 10-50 nucleotides or base pairs, or 15-30 nucleotides or base pairs. In some embodiments, the RNAi is modified, for example, by incorporating non-naturally occurring nucleotides. In some embodiments, the RNAi is a double-stranded RNAi. Suitable lengths of RNAi include, but are not limited to, about 5 to about 200 nucleotides, or 10-50 nucleotides or base pairs, or 15-30 nucleotides or base pairs. In some embodiments, the RNAi is modified, for example, by incorporating non-naturally occurring nucleotides.

In one aspect of the present invention, the RNAi in the library is formed by sequentially connecting a plurality of RNAi units. In one aspect of the present invention, the RNAi in the RNAi molecule library is obtained by sequentially connecting and synthesizing a plurality of RNAi units. The number m of groups of the RNAi units is an integer of 3-10, preferably an integer of 4-8, for example, n is 4 or 5 or 6, which is the same as the number n of amino acid residue units in the polypeptide barcode tag on the corresponding bead. The RNAi unit may be a fragment of its sequence. Each RNAi unit may have different sequence differences or nucleoside modifications, thereby forming RNAi molecules with different sequences or chemical modifications.

In another aspect of the present invention, the nucleic acid molecule in the library is mRNA.

In one aspect of the present invention, the mRNA in the library is formed by sequentially connecting multiple mRNA units. In one aspect of the present invention, the mRNA in the mRNA molecule library is obtained by sequentially connecting and synthesizing multiple mRNA units. The number of groups m of the mRNA unit is an integer of 3-10, preferably an integer of 4-8, for example, n is 4 or 5 or 6, which is the same as the number n of amino acid residue units in the polypeptide barcode label on the corresponding bead. In one aspect of the present invention, the mRNA has a 5' cap, a 5' untranslated region, i.e., a 5'UTR, an mRNA coding sequence, a 3' untranslated region, i.e., a 3'UTR, and a polyadenylic acid tail, connected in sequence. In one aspect of the present invention, the mRNA unit may be an mRNA coding sequence or a fragment thereof (a fragment constituting a complete coding sequence), a flanking region of mRNA (5' untranslated region, i.e., 5'UTR or 3' untranslated region, i.e., 3'UTR) and/or a terminal region (such as a 5' cap, a polyadenylic acid tail). For example, the mRNA may be composed of 5 groups of mRNA units, which are respectively composed of a 5' cap, a 5' untranslated region, i.e., 5'UTR, an mRNA coding sequence, a 3' untranslated region, i.e., 3'UTR, and a polyadenylic acid tail of mRNA in order. In the process of constructing an mRNA library, each unit has a different number of unit members, such as 2-200, or 2-100, or 2-50, or 2-20, or 2-10. For example, when the mRNA unit is an mRNA coding sequence fragment, it may be 2-100 sequence fragments with differently modified nucleosides. For another example, when the mRNA unit is a 5' cap, it can be 2-10 different 5' caps or cap analogs. When 10 groups of mRNA units (e.g., 10 fragments of mRNA coding sequences, respectively) are used for synthesis, and each group includes 5 members, a library containing 510 mRNA molecules will be generated. Each bead in the library has multiple copies of one of the mRNA molecules in the library and has a unique polypeptide barcode tag corresponding to the mRNA bound thereto. In one aspect of the present invention, the mRNA unit comprises one or more modifications, for example, wherein the coding region, the flanking region and/or the terminal region may contain one, two or more (optionally different) nucleoside or nucleotide modifications.

The 5' cap is a modified guanine nucleotide attached to the 5' end of the RNA molecule using a 5'-5' triphosphate bond. The term "5' cap" is also intended to encompass 5' cap analogs, including, for example, 5' diguanosine caps, tetraphosphate cap analogs with methylene-bis(phosphonate) moieties, cap analogs with sulfur substitutions for non-bridging oxygens, N7-benzylated dinucleoside tetraphosphate analogs, or anti-reverse cap analogs, etc. In one such embodiment, the 5' cap analog is a 5' diguanosine cap. The 5' cap is important for recognizing mRNA and attaching it to the ribosome to begin translation. The 5' cap protects the modified mRNA or RNAi from 5' exonuclease-mediated degradation. It is not an absolute requirement for the modified mRNA or RNAi molecule to include a 5' cap, so in yet another aspect of the invention, the modified mRNA or RNAi molecule lacks a 5' cap. However, due to the longer half-life and increased translation efficiency of the modified mRNA comprising a 5' cap, the modified RNA comprising a 5' cap in the present invention is preferred.

5' and/or 3' untranslated region (UTR). The non-translated region is the region of RNA in front of the start codon (5') and after the stop codon (3'), and is therefore not translated. Because the non-translated region can interfere with

ribonucleases and other proteins involved in RNA degradation, the modification of RNA molecules with one or more non-translated regions can improve the stability of mRNA. In addition, the modification of RNA with 5' and/or 3' non-translated regions can enhance translation efficiency. The modification of RNA with 3'UTR can be used to maintain the cytoplasmic localization of RNA, allowing translation to occur in the cytoplasm of cells. In another aspect of the present invention, the modified mRNA does not include 5' or 3'UTR. In another aspect of the present invention, the modified mRNA includes 5' or 3'UTR. In another aspect of the present invention, the modified mRNA includes both 5' and 3'UTR. In another aspect of the present invention, 5' and/or 3'UTR are selected from mRNAs known to have high stability in cells (e.g., mouse $\alpha$-globulin 3'UTR). In yet another aspect of the invention, the 5' UTR, 3' UTR, or both comprise one or more modified nucleosides.

In yet another aspect of the invention, the mRNA further comprises a Kozak sequence. The presence of a Kozak sequence near the AUG codon will strengthen the codon as a start site for translation, allowing correct polypeptide translation to occur. In addition, the addition of a Kozak sequence to the modified RNA will promote more efficient translation. Therefore, in yet another aspect of the invention, the modified RNA further comprises a Kozak consensus sequence at the desired site for initiating translation to produce a polypeptide of the correct length. In yet another aspect of the invention, the Kozak sequence comprises one or more modified nucleosides.

In yet another aspect of the invention, the modified mRNA and/or RNAi molecule further comprises a "polyA tail", which refers to a 3' homopolymer tail of adenine nucleotides, the length of which can vary (e.g., at least 5 adenine nucleotides) and can be up to hundreds of adenine nucleotides). Including a 3' polyA tail can protect the modified RNA from degradation within the cell and also promote extranuclear localization to enhance translation efficiency. In some embodiments, the polyA tail comprises between 20 and 300 adenine nucleotides; in other embodiments, the polyA tail comprises at least 20, at least 30, at least 50, at least 100, at least 110, at least 120, at least 130, at least 140, at least 150, at least 160, at least 170, at least 180, at least 190, at least 200, at least 300 adenine nucleotides or more. In yet another aspect of the invention, polyA tail comprises between 20 and 200 adenine nucleotides. In yet another aspect of the invention, polyA tail comprises one or more modified nucleosides.

In one aspect of the present invention, the mRNA or RNAi unit in the library comprises a modification. For example, modifications of mRNA and/or RNAi include, but are not limited to, (a) terminal modifications, such as 5' terminal modifications (phosphorylation, dephosphorylation, conjugation, reverse bond, etc.), 3' terminal modifications (conjugation, DNA nucleotides, reverse bond, etc.), (b) base modifications, such as replacement with modified bases, stable bases, destabilizing bases, or bases that pair with the extended library base of a partner, or conjugated bases, (c) sugar modifications (such as at the 2' position or the 4' position) or replacement of sugars, and (d) internucleoside bond modifications, including modification or replacement of phosphodiester bonds.

In the mRNA and/or RNAi of the present invention, the modified nucleosides may include $N^6$-methyladenosine ($m^6A$), 5-methoxyuridine (5moU), inosine (I), 5-methylcytosine (m5C), pseudouridine ($\Psi$), 5-hydroxymethylcytosine ($hm^5C$), and $N^1$-methyladenosine ($m^1A$), N1-methylpseudouridine (me(1) $\psi$ ), 5-methylcytidine (5mC), 3,2'-O-dimethyluridine (m4U), 2-thiouridine (s2U), 2'fluorouridine, 2'-O-methyluridine (Um), 2'deoxyuridine (2'dU), 4-thiouridine (s4U), 5-methyluridine (m5U), 2'-O-methyladenosine (m6A), N6,2'-O-dimethyladenosine (m6Am), 2'-O-methylcytidine (Cm), 7-methylguanosine (m7G), 2'-O-methylguanosine (Gm), N2,7-dimethylguanosine (m2,7G), and inosine (I), etc.

The RNA for screening in the nucleic acid library of the present invention may have various activities and functions. In one aspect of the present invention, the RNA is an mRNA encoding a protein or a variant thereof. For example, the mRNA encodes an antibody or a fragment thereof.

In one aspect of the present invention, the RNA can be used as a vaccine. In another aspect of the present invention, when introduced into mRNA, certain modified nucleosides or combinations thereof will activate the innate immune response. When combined with polypeptides and/or other vaccines, such activated molecules can be used as adjuvants. In another aspect of the present invention, the activated molecule contains a translatable region that encodes a polypeptide sequence that can act as a vaccine, thereby providing the ability to become a self-adjuvant. In another aspect of the present invention, the mRNA can encode an immunogen. Delivery of mRNA encoding an immunogen can activate an immune response. The mRNA of the present invention may encode a polypeptide sequence of a vaccine and may further include an inhibitor. An inhibitor may weaken and/or inhibit antigen presentation.

In one aspect of the present invention, the RNA may be used as a therapeutic agent. In one aspect of the present invention, the mRNA encodes a therapeutic protein. In another aspect of the present invention, the protein encoded by the mRNA may increase the natural amount of the protein in a cell. In another aspect of the present invention, the mRNA expresses a naturally occurring protein or a variant thereof with improved disease-modifying activity (including increased biological activity, improved patient outcomes or protective functions, etc.).

In one aspect of the present invention, the nucleic acid molecules or small molecule compounds in the library have a cleavable linker that is releasably connected to the bead. Optionally, the cleavable linker is selected from: a light-cleavable linker, a temperature-cleavable linker, a pH-sensitive linker, an acid-cleavable linker, a base-cleavable linker, a sound-cleavable linker, a salt-cleavable linker, a redox-sensitive linker, or a physically cleavable linker; and a combination of two or more of the foregoing. In one aspect of the present invention, the cleavable linker is a UV-cleavable linker.

The present invention also provides a method for screening the above-mentioned polypeptide encoded nucleic

acid library or polypeptide encoded small molecule compound library. The method for screening the polypeptide encoded nucleic acid library or polypeptide encoded small molecule compound library provided by the present invention is particularly suitable for high-throughput and rapid screening of libraries containing a large number of nucleic acid molecules or small molecule compounds.

The method for screening the above-mentioned polypeptide encoded nucleic acid library or polypeptide encoded small molecule compound library provided by the present invention includes identifying the screened nucleic acid molecules or small molecule compounds by decoding the corresponding polypeptide barcode tag thereof.

The method for screening the above-mentioned polypeptide encoded nucleic acid library or polypeptide encoded small molecule compound library provided by the present invention includes contacting the nucleic acid molecule or small molecule compound library with a biological target. The biological target includes cells and biologically active molecules, such as proteins, nucleic acids and polysaccharides. The nucleic acids or small molecule compounds in the library involved in the present invention can act on cells and biologically active molecules, and the desired nucleic acids or small molecule compounds can be screened by identifying the results of the action.

In one aspect of the present invention, the nucleic acid or small molecule compound can bind to the biological target. For example, the binding of nucleic acid to protein or nucleic acid, or the binding of small molecules to protein occurs. **In** one embodiment of the present invention, the method for screening the above-mentioned polypeptide encoded nucleic acid library or polypeptide encoded small molecule compound library includes contacting the library with a biological target under conditions suitable for the nucleic acid molecule or small molecule compound of the library to bind to the biological target, optionally including removing library members that do not bind to the target, and analyzing the tags associated with the small molecule nucleic acid molecule or small molecule compound bound to the target.

In one aspect of the present invention, the nucleic acid or small molecule compound can have an effect on cells.

In one embodiment of the present invention, in the method for screening the polypeptide encoded nucleic acid library or polypeptide encoded small molecule compound library, the library includes a plurality of beads, each bead includes a polypeptide barcode tag connected to the bead and a nucleic acid molecule or small molecule compound corresponding to the polypeptide barcode tag, and the nucleic acid molecule or small molecule compound is releasably connected to the bead.

In one embodiment of the present invention, the method comprises the following steps: (a) providing the library comprising a plurality of beads having different polypeptide barcode tags and corresponding nucleic acid molecules or small molecule compounds; (b) releasing the nucleic acid molecules or small molecule compounds of each bead from the bead to produce a plurality of free nucleic acid molecules or small molecule compounds; (c) contacting the nucleic acid molecules or small molecule compounds of each bead with a single or multiple biological targets (e.g., cells) to screen the nucleic acid molecules or small molecule compounds to produce a plurality of different screened nucleic acid molecules or small molecule compounds; (d) identifying the screened nucleic acid molecules or small molecule compounds by decoding the polypeptide barcode tags associated therewith.

Cell has the common meaning used in biology, for example, cell refers to an autonomous self-replicating unit that can exist as a functionally independent unit of life (e.g., a unicellular organism) or as a subunit of a multicellular organism (e.g., in plants and mammals). However, cell can also refer to a dormant cell, which is generally still capable of cell division when a mitotic stimulus is applied. In one aspect of the present invention, cell refers to a prokaryotic cell or a eukaryotic cell, preferably a eukaryotic cell. Eukaryotic cells include: mammalian cells, plant cells and fungal cells, etc., preferably mammalian cells.

Mammals refer to any mammals, including humans, livestock and farm animals, as well as zoo, sports or pet animals, such as dogs, cats, cows, horses, sheep, pigs, goats, rabbits, etc. Preferably, the mammal is a human.

In one aspect of the present invention, a method for screening a polypeptide encoded nucleic acid library is provided, the library comprising a plurality of beads, each bead comprising a polypeptide barcode tag connected to the bead and a nucleic acid molecule corresponding to the polypeptide barcode tag, the nucleic acid molecule being releasably connected to the bead.

The method comprising the following steps: (a) providing the library comprising a plurality of beads having different polypeptide barcode tags and corresponding nucleic acid molecules; (b) releasing the nucleic acid molecule of each bead from the bead to produce a plurality of free nucleic acid molecules; (c) contacting the nucleic acid molecule of each bead with a single or multiple biological targets (e.g., cells) to screen the nucleic acid molecules to produce a plurality of different screened nucleic acid molecules; (d) identifying the screened nucleic acid molecules by decoding the polypeptide barcode tags associated therewith.

In one aspect of the present invention, step (d) includes a step of decoding the encoded tag by sequencing the polypeptide barcode tag. In one aspect of the present invention, the sequencing is performed by mass spectrometry, and the information of the polypeptide barcode is read out by mass spectrometry of the sample.

In one aspect of the present invention, step (d) also includes a step of removing the polypeptide barcode tag from the bead before mass spectrometry sequencing of the polypeptide barcode tag.

In one aspect of the present invention, the nucleic acid molecules in the library are releasably connected to the

bead through a cleavable linker. Optionally, the cleavable linker is selected from: a photocleavable linker, a temperature cleavable linker, a pH-sensitive linker, an acid cleavable linker, a base cleavable linker, a sound cleavable linker, a salt cleavable linker, a redox-sensitive linker or a physically cleavable linker; and a combination of two or more of the foregoing. In one aspect of the present invention, the cleavable linker is an ultraviolet cleavable linker.

In one aspect of the present invention, step (b) includes releasing each nucleic acid molecule from the bead under conditions where the cleavable linker is cleavable to produce a plurality of free nucleic acid molecules.

The above-mentioned method for screening a polypeptide encoded nucleic acid library or a polypeptide encoded a small molecule compound library provided by the present invention is suitable for using a microfluidic method and being performed in a microfluidic device. Microfluidics generally refers to a channel in which the size of the fluid circulates is less than 1 mm and includes, for example, from 1 μm to 1 mm. Microfluidic methods generally refer to methods of manipulating a small amount of fluid (10-4 to 10-18 liters) using a microfluidic channel with a size less than 1 mm. Microfluidic devices contain microfluidic channels, and their structural forms include microfluidic devices and microfluidic chips, etc.

In one aspect of the present invention, step (a) of the method includes encapsulating different beads of the library in a plurality of first discrete entities. In one aspect of the present invention, a single one of the plurality of first discrete entities statistically contains one bead.

In one aspect of the present invention, step (c) of the method includes encapsulating the biological target (e.g., cell) in a plurality of second discrete entities. In one aspect of the present invention, a single one of the plurality of second discrete entities statistically contains one cell or a desired number of cells. Multiple cells may be advantageous for observing the signals generated by the effects of the nucleic acid molecules or small molecules to be screened. A plurality of cells generally refers to 2 to about 50, or 2 to about 16, or 2 to 8 or 2 to 4 cells. In one aspect of the present invention, a plurality of cells is obtained by culturing a single cell in a discrete entity. In another aspect of the present invention, a discrete entity containing a plurality of cells is obtained by fusing a plurality of discrete entities containing a single cell.

The term "discrete entity" refers to an emulsion, well, compartment and container capable of encapsulating and/or containing a bead connected to a polypeptide encoded nucleic acid or a small molecule as in the library involved in the present invention, or encapsulating and/or containing the one or a specified number of multiple cells. In one aspect of the present invention, the discrete entity is a droplet. The discrete entity may be spherical or any other suitable shape. The discrete entity has a certain size, for example, a diameter of about 1.0 μm to 1000 μm, such as 1.0 μm to 500 μm, 1.0 μm to 100 μm, 1.0 μm to 10 μm or 1.0 μm to 5 μm. The discrete entities may be generated within, on or through a microfluidic device and/or may flow from or be applied through a microfluidic device.

In one aspect of the invention, the discrete entities as described herein are droplets. The terms "droplet" and "droplet" are sometimes used interchangeably herein to refer to small, generally spherical structures containing at least a first fluid phase, such as an aqueous phase (e.g., water), surrounded by a second fluid phase (e.g., oil) that is immiscible with the first fluid phase. In some embodiments, the second fluid phase will be an immiscible phase carrier fluid. In the present invention, the droplets are preferably water-in-oil emulsions, which are advantageous for encapsulating cells, nucleic acids, enzymes, reagents, and various other components and for culturing or reacting therein.

A wide range of different emulsification methods are well known to those skilled in the art, any of which can be used to produce the droplets of the present invention. Many emulsification techniques involve mixing two liquids in bulk, typically using turbulence to enhance droplet breakup. Such methods include vortexing, sonication, homogenization, or a combination thereof.

The size of the droplets follows a probability distribution, such as a Gaussian distribution. It will also be further understood that the parameters used to prepare the microfluidic droplets can be selected to obtain a plurality of microfluidic droplets having a specific volume. Preferably, the droplets of the present invention have substantially the same shape and/or size. The volume or size of a droplet refers to the average volume or size of a plurality of droplets. A person skilled in the art will be able to determine the average diameter of a population of droplets, for example using laser scattering or other known techniques. The droplets may be spherical or, in some cases, non-spherical. The diameter of a droplet (particularly a non-spherical droplet) may be the diameter of a perfect mathematical sphere having the same volume as a non-spherical droplet.

In one aspect of the present invention, a single bead of the library is encapsulated in a single droplet. In one aspect of the present invention, a single or desired number of multiple biological targets such as cells are encapsulated in a single droplet. In one aspect of the present invention, a single bead is encapsulated with a single or desired number of multiple biological targets such as cells and other reaction reagents in a single droplet. In one aspect of the present invention, a single bead or a single or desired number of multiple biological targets such as cells and other reaction reagents are encapsulated in a single droplet.

Various known methods can be used to controllably encapsulate particles such as beads or cells into droplets. For example, PCT/EP2016/080341 describes techniques for encapsulating cells in microfluidic droplets. A person skilled in the art can easily derive techniques for preparing droplets containing other kinds of particles and/or containing reagents dissolved in solution from the same disclosure (e.g. PCT/EP2016/080341).

In one aspect of the present invention, particles such as beads or cells can be controllably encapsulated into

droplets using, for example, inertial sorting. Particles such as beads or cells flow through a channel at high speed, causing inertial effects to become important and inertial sorting of particles in the channel, resulting in periodic spacing of particles. The period of the liquid flow containing the particles can then be matched to the droplet generation period of the droplet maker, so that the particles may be effectively encapsulated in the droplets.

In one aspect of the present invention, the microfluidic device used in the method of the present invention for screening a polypeptide encoded nucleic acid or a small molecule compound library includes a droplet generation unit for preparing droplets containing beads and/or reagents dissolved in solution containing biological targets such as cells or libraries. The droplet generation unit typically includes an inlet for a droplet carrier oil (carrier fluid), and an additional inlet for a droplet aqueous phase component. For the carrier oil, typically, a fluorinated oil (e.g., HFE-7500) containing, for example, 0.75% (w/w) surfactant (PFPE-PEG-PFPE triblock copolymer containing two perfluoropolyether blocks (PFPE) and one polyethylene glycol (PEG) block) is typically used. Surfactants are typically used to prevent droplet coalescence, and their dosage can be adjusted, for example, based on the physicochemical properties of the surfactant used. The carrier oil used for emulsification is not limited to fluorinated liquids, and alternative fluids such as hydrocarbon-based fluids (e.g., mineral oil, hexane, etc.), silicone oils, and other types of oils can be successfully used. For the fluid for preparing the beads of a single cell/single library, typically one inlet is used for the droplet carrier oil, and typically there is another inlet for delivering a suspension of beads of the cell/single library. For the preparation of droplets containing reagents dissolved in a solution, one inlet is usually used for the droplet carrier oil, and one or more inlets deliver different reagents. In addition, there may be an inlet for delivering a suspension of cells/beads of a single library.

In one aspect of the present invention, in step (c) of the method for screening a library of polypeptide-encoded nucleic acids or small molecule compounds, the contact of the nucleic acid molecule or small molecule compound of each bead of the library with a single or a desired number of multiple biological targets such as cells is limited within a microcompartment.

The microcompartment is physically limited to the physical proximity of the nucleic acid molecule or small molecule compound to a single or a desired number of multiple cells (for example, the cell is exposed within 1000 $\mu$m of each nucleic acid molecule or small molecule compound) and isolated from other nucleic acid molecules or small molecule compounds or cells. Microcompartments are implemented including microfluidic channels, droplets, microparticles, micropores and microbubbles. In the present invention, the preferred microcompartment is a droplet.

In one aspect of the present invention, the method includes encapsulating a single bead of the library and a single cell or a desired number of multiple biological targets such as cells in a single microcompartment. In one aspect of the present invention, the method comprises encapsulating a single bead of the library and a single biological target such as a cell or a desired number of multiple biological targets such as cells and other reagents (such as reagents for detecting biological responses caused by the nucleic acid molecule or small molecule compound in the cell) in a single microcompartment.

In one aspect of the present invention, the method comprises encapsulating a single bead of the library and a single biological target such as a cell in a single discrete entity such as a droplet. In one aspect of the present invention, the method comprises encapsulating a single bead of the library and a desired number of multiple biological targets such as cells in a single discrete entity such as a droplet. In another aspect of the present invention, the method comprises encapsulating a single bead of the library and a single biological target such as a cell or a desired number of multiple biological targets such as cells and other reagents (such as reagents for detecting biological responses caused by the nucleic acid molecule or small molecule compound in the cell) in a single droplet.

In one aspect of the present invention, a solution (such as an aqueous solution) containing beads of the library or the biological target such as cells is introduced through two intersecting microfluidic channels, and an emulsion droplet containing beads of the library or the biological target such as cells is formed at the interface with the introduced carrier liquid (such as oil). In another aspect of the present invention, other reagents (such as reagents for detecting biological responses caused by the nucleic acid molecules or small molecule compounds in cells) can be added to the above-mentioned solution (such as an aqueous solution) containing beads of the library or the cells. In another aspect of the present invention, a solution containing other reagents (such as reagents for detecting biological responses caused by the nucleic acid molecules or small molecule compounds in cells) is introduced through a microfluidic channel other than the solution (such as an aqueous solution) containing beads of the library or the biological target such as cells through two intersecting microfluidic channels.

In one aspect of the present invention, the method includes encapsulating a single bead of the library and a single biological target such as a cell in a single discrete entity such as a droplet. In one aspect of the present invention, a single bead of the library is encapsulated in a single first droplet, and the single or desired number of multiple biological targets such as cells are encapsulated in a single second droplet, and then the first droplet containing the beads and the second droplet containing the cells are merged to form a droplet. In another aspect of the present invention, in the above method, the single bead and/or a single or desired number of multiple biological targets such as cells and other reaction reagents (such as reagents for detecting the biological response caused by the nucleic acid molecule or small molecule compound in the cell) are encapsulated in a single droplet.

In one aspect of the present invention, the method includes the step of forming a one-to-one paired combination of the first droplet and the second droplet. **In** another aspect of the present invention, the method includes the step of merging the one-to-one paired combination of the first droplet and the second droplet. **In** one aspect of the present invention, a merged single droplet is obtained by applying an electric field to the paired combination of the first droplet and the second droplet.

Various methods known in the art can be used to form a one-to-one paired combination of the first droplet and the second droplet. For example, the first droplet and the second droplet can be added separately through two intersecting microchannels, and the size and flow rate of the microchannel can be controlled to cause orderly periodic spacing of the droplets, and then the period of the flow into the first droplet can be matched with the period of the flow into the second droplet, so that the first droplet and the second droplet form a one-to-one pairing combination in the form of "ABAB" at the channel interface. In another aspect of the present invention, it also includes the step of contacting the first droplet and the second droplet that form a one-to-one pairing to apply an electric field to them. One embodiment is to make the first droplet and the second droplet have different volumes, so that the smaller droplet "catch up" with the larger droplet in the flow channel to form a contacting droplet group. Then, the adjacent droplets can be merged by applying an electric field to them as known in the art.

Fluid droplets can be merged using any suitable technology. For example, in a microfluidic system, the surface tension of the droplets related to the droplet size can prevent the merging or coalescence of the droplets. In one embodiment, two droplets may be given opposite charges (i.e., positive and negative charges, not necessarily of the same magnitude), which may enhance the electrical interaction of the two droplets, allowing merging and coalescence of the droplets to occur. The charge (positive or negative) may be imparted to the droplets by using a Taylor cone or any other suitable technique. For example, an electric field may be applied to a reactor containing droplets, the droplets may pass through a container, a chemical reaction may occur to cause the droplets to be charged; the droplets may flow through regions with opposite wetting properties, and so on.

In one aspect of the present invention, a microfluidic device for preparing beads containing cells or libraries and/or droplets containing reagents dissolved in a solution includes a droplet merging unit for merging two or more droplets. In one aspect of the present invention, the droplet merging unit includes: intersecting flow channels for respectively inputting a first droplet and a second droplet; a flow channel for forming a one-to-one paired first droplet and a second droplet; an electric field for droplet merging that applies an electric field to the paired first droplet and second droplet so that the first droplet and the second droplet merge into a single droplet.

In one aspect of the present invention, the method further comprises the step of sorting a first discrete entity containing the beads or a second discrete entity containing cells from the discrete entities such as droplets. The sorting can be performed by methods known in the art such as fluorescence-activated droplet sorting (FADS) or deterministic lateral displacement (DLD).

The method of the present invention is expected to encapsulate one bead or one biological target such as a cell (or a specified number of cells) in each droplet. However, because discrete targets such as molecules, beads, cells, etc. are usually randomly encapsulated, the encapsulation statics follow a Poisson distribution, resulting in many empty and unusable droplets. When trying to achieve co-encapsulation of two discrete targets such as cells and beads, the vast majority of droplets produced will be empty or contain a single cell or bead, and a very small portion will contain both cells and beads. One way to overcome this inefficiency is to perform sorting.

Fluorescence-activated droplet sorting (FADS) is an adjustment of the mature fluorescence-activated cell sorting (FACS) equipment and protocols to sort the generated droplets. For example, described in Baret et al. (2009) Lap Chip 9:1850-1858. Any change that can be detected by using a fluorescent moiety can be screened. Target cells can be fluorescently labeled to enable FADS. A variety of fluorescent proteins can be used as labels for this purpose. In addition, a very wide range of dyes that fluoresce at specific levels and conditions within cells can be used. Examples include those purchased from Molecular Probes (Thermo Scientific). In one aspect of the invention, antibodies can be used to detect cellular components, which can be stained with any number of fluorophores using commercially available kits.

DLD is provided in a microfluidic device by providing a columnar matrix, and the fluid streamlines between two adjacent columns are divided into several parts. For large particles, because the particle diameter is greater than the critical sieving size, its motion trajectory will be laterally offset along the offset direction of the column array; for small particles, it will move along the fluid streamlines close to the column, and the direction of movement is basically unchanged, that is, it moves forward along a straight line. Large and small particles enter the flow channel together and move forward in different directions. After a certain screening distance, large and small particles (such as droplets containing target particles and droplets without "empty bag" particles) are located on both sides of the flow channel in the downstream flow channel.

In the polypeptide encoded nucleic acid or small molecule compound library provided by the present invention or the library analyzed by the aforementioned method of the present invention, beads with different polypeptide encoded nucleic acids or small molecule compounds are numerous. The number is, for example, $>10^4$; or $>10^5$; or $>10^6$; or $>10^7$; or $>10^8$; or $>10^9$; or $>10^{10}$; or $>10^{11}$. The present invention also provides a discrete entity library obtained by encapsulating

the beads of the library into a single discrete entity such as a droplet (with or without sorting), which includes a large number of discrete entities such as droplets containing different beads (with different polypeptide encoded nucleic acids or small molecule compounds), the number of which is, for example, $>10^4$; or $>10^5$; or $>10^6$; or $>10^7$; or $>10^8$; or $>10^9$; or $>10^{10}$; or $>10^{11}$.

In the discrete entity library provided by the present invention, or in the screening method provided by the present invention, since the nucleic acid molecules or small molecules and cells are confined in discrete entities or microcompartments, they can be present at a sufficiently high concentration for action. The concentration is, for example: less than 1nM; 1 to 100nM; greater than 100nM; less than 1μM; 1 to 100μM; greater than 100μM; 5 to 50μM or 10 to 20μM.

In one aspect of the present invention, in step (b) of the screening method, the free nucleic acid molecules or small molecule compounds are released into the first discrete entity. In the present invention, each nucleic acid molecule is released from the bead under conditions where the cleavable linker can be cleaved to produce a plurality of free nucleic acid molecules. In one aspect of the present invention, the polypeptide barcode tag is retained on the bead.

In one aspect of the present invention, in step (c) of the screening method, the free nucleic acid molecule or small molecule compound (e.g., by diffusion) contacts the biological target, for example, enters the cell. In the method of the present invention, various methods known in the art for promoting the permeability of cells to extracellular nucleic acids or small molecules can be used to promote the free nucleic acid molecule or small molecule compound (e.g., by diffusion) to enter the cell. For example, the cell membrane can be temporarily perforated by electroporation or spatial squeezing to promote the free nucleic acid molecule or small molecule compound to enter the cell.

The method of the present invention is used to screen a library of polypeptide-encoded nucleic acids or small molecule compounds to obtain drugs, reagents, sensors or materials with desired properties and functions such as better properties. In one aspect of the present invention, the screening method is used to obtain nucleic acids with better biological functions, especially RNA, such as mRNA and RNAi, which are used for therapeutic or preventive drugs or vaccines.

In one aspect of the present invention, step (c) of the screening method includes incubating cells to allow them to produce biological responses caused by the nucleic acid molecule or small molecule compound. In the present invention, the biological response may be the expression of the target protein or the increase or decrease of the expression, or the stability of the target protein. The target protein may be a protein encoded by the mRNA of the bead or a protein in the cell affected by the protein encoded by the mRNA of the bead. In the present invention, the biological response may also be the survival or death of the cell, or the binding or neutralization of the virus contained in the cell.

In one aspect of the present invention, the assay may detect the morphological response of the cell, such as migration, as well as the genetic response and the biochemical response. The method of the present invention is used to detect biological responses that can generate signals.

In one aspect of the present invention, step (c) in the method is performed in a microchamber (e.g., a droplet), and the microchamber contains a reagent for detecting the biological response caused by the nucleic acid molecule or small molecule compound. The signal entity may include, but is not limited to, fluorescent dyes, chemiluminescent entities, radioactive labels, isotopes such as non-radioactive isotopes or isotopes detectable by mass spectrometry (e.g., charged mass label EML), ligands that can be used as specific binding partners of labeled antibodies, enzymes, antibodies that can be used as specific binding partners of labeled ligands, antigens, groups with specific reactivity, and/or electrochemically detectable parts. Non-limiting examples of fluorescent signal entities include fluorescein, rhodamine, or hexachlorofluorescein, etc.

In one aspect of the present invention, the reagent may be a fluorescent signal generated by detecting the biological response. For example, the reagent may include an antibody that can recognize a specific protein. For example, the reagent may label a fluorescent signal entity, as well as a quencher or enhancer. Non-limiting examples of applicable fluorescent entities include 6-carboxyfluorescein and tetrachlorofluorescein. The quencher or enhancer may be any entity that can affect the signal entity in a certain way, such as by inhibiting or facilitating the determination of the signal entity, respectively. For example, the proximity of a fluorescent signal entity and a quencher may enable the quencher to partially or completely suppress the fluorescence of the signal entity, while the enhancer may be able to enhance the fluorescence of the fluorescent signal entity when the enhancer is located near the signal entity.

In the present invention, the method of determining the marker component and/or signal entity will depend on the components present in the fluid droplet. As described above, techniques such as radioactivity, fluorescence, phosphorescence, light scattering, light absorption, fluorescence polarization, etc. can be used for determination. Many detectors operating using these principles are commercially available. The detector can be used to determine at least one signal entity and/or marker component that may be present in the fluid droplet, and in some cases, more than one detector can be used.

In some embodiments, the droplet is deformed so that the signal entity and/or marker component contained in the droplet passes through the detector in a single file. In some embodiments, detection can be performed in parallel, that is, many signal entities and/or marker components can be determined simultaneously in one channel and/or multiple channels. For example, a timing device can be used to synchronize the detection of parallel pathways. Another non-limiting example of parallel detection is the use of a camera or other imaging device that is arranged to be able to capture more than one channel, for example, simultaneously. The camera can be, for example, a line scan camera, a 2D CCD camera, etc.

In the present invention, the response to a candidate nucleic acid molecule or a small molecule compound can be evaluated by one or more biomarkers. Biomarkers include diagnostic biomarkers, biomarkers that predict whether a particular patient will respond (become better) to a particular drug, and biomarkers that predict whether a particular patient will have unacceptable toxicity to a particular drug. The biological response also includes cytokine expression. The response can be evaluated by measuring expressed cytokines, such as IL-2, IL-4, IL-6, IL-10, IFN-$\gamma$, and TNF-$\alpha$.

In the method of the present invention, after observing particles of cells with desired signals, the particles are sorted. Similarly, fluorescence activated droplet sorting (FADS) can be used to sort the required droplets. In one aspect of the present invention, step (d) in the method includes the step of collecting the screened droplets. In one aspect of the present invention, step (d) in the method includes the step of breaking the collected droplets and collecting beads.

The present invention also provides a microfluidic device for a method for screening a polypeptide encoded nucleic acid or a small molecule compound library. In one aspect of the present invention, the microfluidic device is suitable for screening the polypeptide encoded nucleic acid library or polypeptide encoded small molecule compound library of the present invention.

The microfluidic device provided by the present invention generally includes one or more of the following units:

droplet generation unit, used to prepare beads containing biological targets (such as cells) or polypeptide encoded nucleic acid libraries or polypeptide encoded small molecule compound libraries and/or droplets containing reagents dissolved in solution;
droplet merging unit, used to merge two or more droplets;
biological target response unit, used to allow nucleic acid libraries or polypeptide encoded small molecule compound libraries to contact biological targets and generate biological responses caused by the nucleic acid molecules or small molecule compounds; or
optionally, droplet sorting device.

In one aspect of the present invention, the biological target response unit is a cell culture unit, used to culture cells to allow them to generate biological responses caused by the nucleic acid molecules or small molecule compounds.

In one aspect of the present invention, the droplet generation unit includes: an inlet for droplet carrier oil (carrier fluid) and another inlet for droplet aqueous phase components, and optionally, an inlet for adding other reagents (such as reagents for detecting biological responses caused by the nucleic acid molecules or small molecule compounds in cells).

In one aspect of the present invention, the droplet merging unit includes: intersecting flow channels for respectively inputting the first droplet and the second droplet; a flow channel for forming a one-to-one pairing of the first droplet and the second droplet; and an electric field for droplet merging for applying an electric field to the paired first droplet and the second droplet so that the first droplet and the second droplet merge into a single droplet.

In one aspect of the present invention, the cell culture unit includes a temperature control device.

In one aspect of the present invention, the sorting device is a fluorescence-activated droplet sorting device or a deterministic lateral offset device.

In one aspect of the present invention, the microfluidic device has a cell processing unit for treating cells to promote the permeability of cells to extracellular nucleic acids or small molecules. For example, the cell processing unit has a structure for implementing electroporation or spatial squeezing to produce transient perforations in the cell membrane.

In one aspect of the present invention, the microfluidic device has an ultraviolet light emitting device.

In one aspect of the present invention, the microfluidic device includes a mass spectrometry device.

## Brief Description of the Drawings

In the following the preferred embodiments of the present invention will be described in detail with reference to the accompanying drawings. It is obvious to the skilled in the art that these drawings are only for reference and there are other embodiments of the present invention.

FIG. 1 is a schematic diagram of the beads in the RNA library provided by the present invention.
FIG. 2 is an exemplary flow chart of the present invention using a microfluidic device to screen a polypeptide encoded RNA library.
FIG. 3 is a schematic diagram of an exemplary method and apparatus for forming a droplet containing a single cell.
FIG. 4A and FIG. 4B are schematic diagrams and results, respectively, of exemplary screening of droplets containing a single mRNA library bead using the DLD method.

FIG. 5 is an exemplary schematic diagram of re-injecting a first droplet containing a single bead and a second droplet containing a single cell.

FIG. 6 is a schematic diagram of applying an electric field to a droplet by electroporation to cause a transient perforation of the cell membrane and promote the entry of free mRNA nucleic acid molecules into the cell.

FIG. 7 is an exemplary result diagram of an incubation zone of a droplet containing mRNA library beads and cells.

FIG. 8 is a diagram showing the mRNA sequence encoding G-CSF.

Detailed Description of Embodiments

In order to make the purpose, technical scheme and advantages of the embodiments of the present invention clearer, the technical scheme in the embodiments of the present invention will be clearly and completely described below in conjunction with the drawings in the embodiments of the present invention. Obviously, the described embodiments are part of the embodiments of the present invention, but not all of the embodiments. Based on the embodiments in the present invention, all other embodiments obtained by ordinary technicians in the field without creative work belong to the protection scope of the present invention.

Example 1 Construction of polypeptide encoded mRNA library

This experiment provides a method for a polypeptide encoded mRNA library, the library includes a plurality of beads, each bead includes a polypeptide barcode label connected to the bead and an RNA molecule corresponding to the polypeptide barcode label, and the RNA molecule is releasably connected to the bead.

FIG. 1 is a schematic diagram of a bead in an exemplary RNA library provided by the present invention. As shown in the figure, there are RNA molecules and molecular barcodes on the surface of the bead, and the molecular barcode is a polypeptide. The RNA can be mRNA. When it comes to a small molecule compound library, there are small molecule compounds on the surface of the bead.

In the present invention, the polypeptide barcode label is formed by sequentially connecting n amino acid residue building units. Wherein n is an integer of 3-10, preferably an integer of 4-8, for example, n is 4 or 5 or 6. Each amino acid residue building unit may be composed of one or more amino acid residues, for example, each amino acid residue building unit comprises one or two or four amino acid residues. In the present invention, the RNA molecule is formed by m nucleic acid units connected in sequence, wherein m is the same as the number n of amino acid residue building units in the polypeptide barcode tag. Each nucleic acid unit and each amino acid residue building unit are assembled sequentially and alternately onto the beads. The nucleic acid units and amino acid residue building units are assembled sequentially and alternately, so that the polypeptide barcode label formed on each bead by the multiple amino acid residue building units is unique, and at the same time forms a corresponding relationship with the nucleic acid molecule formed by the multiple nucleic acid units on the same bead: according to the sequence information of the polypeptide barcode label, the composition information of the corresponding nucleic acid molecule can be obtained, including the nucleic acid sequence and nucleoside types determined by the above multiple nucleic acid units.

In the present invention, the number of different nucleic acid molecules contained in the library is determined by the number of groups of nucleic acid units and the number of members in each group of nucleic acid units, and the number of nucleic acid molecules increases exponentially with the number of synthesis steps (determining the number of groups of nucleic acid units) and building blocks (i.e., the number of members in each group of nucleic acid units). This strategy enables the number of nucleic acid molecules in the library to reach the scale of hundreds of millions or even hundreds of billions. For example, when 4 groups of mRNA units (e.g., 5' untranslated region, i.e., 5'UTR, mRNA coding sequence, 3' untranslated region, i.e., 3'UTR, and poly A tail, of mRNA are used in order), and each group includes 10 members for synthesis, a library containing $10^4$ mRNA molecules will be generated. For another example, when 10 groups of mRNA units (e.g., 10 fragments of mRNA coding sequences, respectively) are used, and each group includes 5 members for synthesis, a library containing $5^{10}$ mRNA molecules will be generated. Each bead in the library has multiple copies of one of the mRNA molecule libraries and has a unique polypeptide barcode tag corresponding to the mRNA bound thereto. According to the sequence information of the polypeptide barcode tag of each bead, the composition information of the corresponding nucleic acid molecule can be obtained, including the nucleic acid sequence and nucleoside types determined by the above multiple nucleic acid units.

In this embodiment, a full-length mRNA library of polypeptide-encoded granulocyte colony stimulating factor (G-CSF) was prepared by the following method, and the mRNA had mRNA units connected in the following order: 5' cap and 5' untranslated region, i.e. 5'UTR, mRNA coding sequence or fragment thereof, 3' untranslated region, i.e. 3'UTR, and polyadenylic acid tail, i.e. polyA.

Materials and reagents: Fmoc-L-Asp(OAll)-Wang Resin (0.8mmol/g, 100-200 mesh, 1%DVB), amino acids 1-20 for the label (20 Fmoc-protected carboxylic acids) were purchased from Jier Biochemical (Shanghai) Co., Ltd., piperidine, DCC, HOBt, HATU, HOAt, All-hydroxyethyl photocoupler crosslinker, and trifluoroacetic acid were purchased from

SIGMA. Other chemical reagents were purchased from Inokai Chemical Reagent Company.

Step 1. Functionalization of resin

Silanization of peptide reaction tube: Pour 10% trimethylsilyl chloride-toluene solution into the reaction tube and leave overnight. Filter (the filtrate can be reused). After treatment, the resin will not stick to the reaction tube wall. Add 803 mg of Fmoc-L-Asp(OAll)-Wang Resin (0.8mmol/g, 100-200 mesh, 1%DVB) to the peptide synthesis tube, add 10 mL of dichloromethane, blow nitrogen and stir, swell the resin for 5 minutes, filter DCM, and add 10 mL DMF to wash once. After fully swelling with acetonitrile, add 10mM $PhSiH_3$, $Pd(PPh_3)_4$, and 10ml DCM. After 30 minutes, filter and remove the solution, wash with acetonitrile, add 10 mL of dichloromethane, blow nitrogen and stir, swell the resin for 5 minutes, filter DCM, and add 10 mL DMF to wash once. Repeat this washing three times. Add 0.3 g of THP-hydroxyethyl photocoupler crosslinker (1.0 mmol), anhydrous acetonitrile, DCC, and HOBt, and shake the reaction at room temperature for 24 hours. Filter the solution, wash with acetonitrile, and then wash with acetonitrile to obtain a photosensitive functionalized resin.

Step 2. Immobilization of tag amino acid 1

Add 10 mL of 20% piperidine-DMF solution to the tube, stir with nitrogen for 5 min, filter, and then add 10 mL of 20% piperidine-DMF solution, stir with nitrogen for 20 min, and filter. Add 10 mL of DCM, stir with nitrogen for 1 min, and filter. Add 10 mL of DMF to each reaction tube, stir with nitrogen for 1 min, and filter. Repeat the washing three times. Divide the resin into 5 equal parts and place them in different reactors. Add 1.5 mmol (3 times the amount of resin) of amino acids 1 to 5 (D, S, E, G, H) respectively, add 1.5 mmol HOBTand 1.5 mmol HBTU to each tube in turn, add 10 mL DMF to each tube, shake and dissolve, and finally add 300 μL of DIPEA to each tube, shake well, activate the carboxyl group for 5 minutes, pour into reaction tubes 1 to 5, and react with nitrogen for 1 hour. Filter. Add 10 mL DCM, stir with nitrogen for 1 min, filter; add 10 mL DMF to each reaction tube, stir with nitrogen for 1 min, filter. Repeat the washing three times. Use a capillary to stick a small amount of resin (30 resin balls are enough) into a 1.5 mL centrifuge tube, add a drop of 5% ninhydrin-ethanol solution (99.9% anhydrous ethanol), and heat in a metal reaction bath at 110 °C for 3 min. If the resin ball is colorless (the resin ball is slightly red when connected to Fmoc proline) and the solution is light yellow, the reaction is complete. If the resin ball or solution is light blue, the reaction may not be complete. Add the above-mentioned reaction reagents and repeat the process until the reaction is complete.

Step 3. Immobilization of 5'-cap-5'-UTR fragment

Using 5'-UTR of human α-globin with Kozak sequence: 5 5'-UTR sequence members with sequences as shown below were introduced:

1) GAGAATAAACTAGTATTCTTCTGGTCCCCACAGACTCAGAGAGAACCCGCCACCATG
2) GAGAATAAACTAGTATTCTTCTGGTCCCCACAGACTCAGAGAGAACCCGCCGCCATG
3) GAGAATAAACTAGTATTCTTCTGGTCCCCACAGACTCAGAGAGAACCCGTTACCATG
4) GAGAATAAACTAGTATTCTTCTGGTCCCCACAGACTCAGAGAGAACCCGCCATTATG
5) GAGAATAAACTAGTATTCTTCTGGTCCCCACAGACTCAGAGAGAACCCACCACCATG.

The sequences were provided by Shanghai Jierui Biotechnology Co., Ltd. The synthesized DNA template is used to complete the in vitro transcription and capping reaction (ARCA cap) of the 5'-UTR sequence RNA through in vitro transcription to obtain a 5'-UTR sequence with a 3'-0-Me-m7G(5')ppp(5') cap at 5'.

The above-mentioned capped 5-UTR is dissolved in RNase free water and added to the 5 reactors containing polypeptide tags corresponding to step 2.

After the resin prepared in step 2 is fully swollen with acetonitrile, 10mM $PhSiH_3$, $Pd(PPh_3)_4$, and 10ml DCM are added. After 30 minutes, the solution is removed by filtration, washed with acetonitrile, and 10 mL of dichloromethane is added respectively. Stir with nitrogen. After swelling the resin for 5 minutes, DCM is filtered and 10 mL of DMF is added for washing. Repeat the washing three times. The functionalized resins with All (allyl) protection removed were added to the above five 5'-cap-5'-UTRs, and anhydrous acetonitrile, DCC, and HOBt were added in sequence. After capping, the reaction was carried out under nitrogen bubbling and shaking at room temperature for 24 hours.

Step 3 Connection of G-CSF mRNA

The resins in the five reactors were combined into one reactor.

The full-length mRNA of G-CSF was prepared by Shanghai Jierui Biotechnology Co., Ltd., and the coding sequence was based on NCBI Reference Sequence: NM_172220.3 (which sequence is shown in FIG. 8).

The connection of G-CSF mRNA was performed according to the standard operation of T4 RNA Ligase 1. The prepared G-CSF mRNA was denatured at 65 degrees, then added to the reactor and washed three times with RNase free water. 10 × Reaction Buffer, T4 RNA Ligase 1, RNase Inhibitor, ATP (10 mM), PEG8000 were mixed at a ratio of 4:2:1:4:8, added to the reaction system at once, and reacted at 37 degrees with nitrogen bubbling for 30 minutes. Stop the reaction and wash thoroughly with RNase free water 3 times. Complete the connection of G-CSF mRNA.

Step 4. Immobilization of tag amino acid 2

Merge the resins obtained in step 3, add 10 mL of 20% piperidine-DMF solution to the tube, stir with nitrogen for 5 minutes, filter, add 10 mL of 20% piperidine-DMF solution, stir with nitrogen for 20 minutes, filter. Stir with nitrogen for 1 minute, filter; add 10 mL of DMF to each reaction tube, stir with nitrogen for 1 minute, filter. Repeat the washing three times. Divide the resin into 5 parts and place them in different reactors. Add 1.5 mmol (3 times the amount of resin) of amino acids 6 to 10 (R, T, A, P, C) respectively, add 1.5 mmol HOBT and 1.5 mmol HBTU to each tube in turn, add 10 mL DMF to each tube, shake and dissolve, and finally add 300 μL of DIPEA to each tube, shake well, activate the carboxyl group for 5 minutes, pour into reaction tubes 1 to 5, and react with nitrogen for 1 hour. Filter. Add 10 mL DCM, stir with nitrogen for 1 min, filter; add 10 mL DMF to each reaction tube, stir with nitrogen for 1 min, filter. Repeat the washing three times. Use a capillary to stick a small amount of resin (30 resin balls are enough) into a 1.5 mL centrifuge tube, add a drop of 5% ninhydrin-ethanol solution (99.9% anhydrous ethanol), and heat in a metal reaction bath at 110 °C for 3 min. If the resin ball is colorless (the resin ball is slightly red when connected to Fmoc proline) and the solution is light yellow, the reaction is complete. If the resin ball or solution is light blue, the reaction may not be complete. Add the above reaction reagents and repeat the process until the reaction is complete.

Step 5. 3'-UTR connection

Using the 3'-UTR sequence of α-globin as a template, adjust some bases to optimize the translation efficiency and introduce 5 3'-UTR sequences, as follows:

1） GCTGGAGCCTCGGTGGCCTAGCTTCTTGCCCCTTGGGCCTCCCCCCAGCCCCTCCTCCCC TTCCTGCACCCGTACCCCGTGGTCTTTGAATAAAGTCTGAGTGGGCGGCA

2） GCTGGAGCCTCGGTGGCCTAGCTTCTTGCCCCTTGGGCCTCCCCCCAGCCCCTCCTAAAA TTCCTGCACCCGTAAAAAGTGGTCTTTGAATAAAGTCTGAGTGGGCGGCA

3） GCTGGAGCCTCGGTGGCCTAGCTTCTTGCCCCTTGGGCCTAAAAAAAGCCCCTCCTAAAA TTCCTGCACCCGTACCCCGTGGTCTTTGAATAAAGTCTGAGTGGGCGGCA

4） GCTGGAGCCTCGGTGGCCTAGCTTCTTGCCCCTTGGGCCTCCCCCCAGCCCCTCCTCCCC TTCCTGCACCCGTAAAAAGTGGTCTTTGAATAAAGTCTGAGTTTTCGGCA

5） GCTGGAGCCTCGGTGGCCTAGCTTCTTGCCCCTTTTTCCTAAAAAAAGCCCCTCCTCCCC TTCCTGCACCCGTACCCCGTGGTCTTTGAATAAAGTCTGAGTTTTCGGCA.

The sequence was prepared by Shanghai Jierui Bioengineering Co., Ltd. The above 3'-UTR was dissolved in RNase free water, added to the 5 reactors containing polypeptide tags corresponding to step 4, and washed 3 times with RNase free water. The 3'-UTR was connected to the mRNA according to the technical scheme of T4 RNA Ligase 1. The specific operation is as follows: 10 x Reaction Buffer, T4 RNA Ligase 1, RNase Inhibitor, ATP (10 mM), PEG8000 were mixed in a ratio of 4:2:1:4:8, added to the reaction system at once, and reacted at 37 degrees with nitrogen bubbling for 30 minutes. Stop the reaction and wash thoroughly with RNase free water 3 times.

Step 6. Immobilization of tag amino acid 3

Merge the resins obtained in step 5, add 10 mL of 20% piperidine-DMF solution to the tube, stir with nitrogen for 5 minutes, filter, add 10 mL of 20% piperidine-DMF solution, stir with nitrogen for 20 minutes, filter, stir with nitrogen for 1 minute, and filter. Repeat the washing three times. Divide the resin into 5 parts and place them in different reactors. Add 1.5

mmol (3 times the amount of resin) of amino acids 11-15 (L, F, W, Q, N) respectively, add 1.5 mmol HOBT and 1.5 mmol HBTU to each tube in turn, add 10 mL DMF to each tube, shake and dissolve, and finally add 300 μL DIPEA to each tube, shake well, activate the carboxyl group for 5 minutes, pour into reaction tubes 1 to 5, and react with nitrogen for 1 hour. Filter. Add 10 mL DCM, stir with nitrogen for 1 min, and filter; add 10 mL DMF to each reaction tube, stir with nitrogen for 1 min, and filter. Repeat the washing three times. Use a capillary to stick a small amount of resin (30 resin balls are enough) into a 1.5 mL centrifuge tube, add a drop of 5% ninhydrin-ethanol solution (99.9% anhydrous ethanol), and heat in a metal reaction bath at 110 ° C for 3 min. If the resin balls are colorless (the resin balls are slightly red when connected to Fmoc proline) and the solution is light yellow, the reaction is complete. If the resin balls or the solution are light blue, the reaction may not be complete. Add the above reaction reagents and repeat the process until the reaction is complete.

Step 7. Poly A tailing

The length of the Poly A tail has a significant effect on the efficiency of mRNA translation and the number of expressions. Usually, a length of 110-150 is more suitable. The present invention uses 5 types of Poly A tails, with lengths of 110, 120, 130, 140, and 150, respectively, all of which are customized by Shanghai Jierui Bioengineering Co., Ltd.

Treat the resin with mRNA at 65 degrees to denature it, and wash it with RNase free water 3 times. Connect the Poly A tail to the mRNA according to the technical scheme of T4 RNA Ligase1. The specific operation is as follows: 10 x Reaction Buffer, T4 RNA Ligase 1, RNase Inhibitor, ATP (10 mM), PEG8000 are mixed at a ratio of 4:2:1:4:8, added to the reaction system at one time, and reacted at 37 degrees with nitrogen aeration for 30 minutes. Stop the reaction and wash thoroughly with RNase free water 3 times.

Thus, a full-length mRNA library of polypeptide-encoded G-CSF is prepared, wherein the mRNA has mRNA units connected in the following order: 5' cap and 5' untranslated region, i.e., 5'UTR, mRNA coding sequence or its fragment, 3' untranslated region, i.e., 3'UTR, and poly A tail, i.e., poly A. The library includes candidate mRNAs of G-CSF full-length coding sequences with 5 different 5' cap-5'UTR, 5 different 3'UTRs, and 5 different polyadenylates, i.e., the library capacity is 5×5×5=125 mRNAs. Each candidate mRNA has a unique polypeptide barcode tag.

Example 2 Screening of mRNA polypeptide encoded library using microfluidic device

The present invention provides a method for screening polypeptide encoded RNA library, which comprises the following steps: (a) providing the library comprising a plurality of beads having different polypeptide barcode labels and corresponding nucleic acid molecules or small molecule compounds; (b) releasing the nucleic acid molecules or small molecule compounds of each bead from the beads to produce a plurality of free nucleic acid molecules or small molecule compounds; (c) contacting the nucleic acid molecules or small molecule compounds of each bead with a single or multiple cells to screen the nucleic acid molecules or small molecule compounds to produce a plurality of different screened nucleic acid molecules or small molecule compounds; (d) identifying the screened nucleic acid molecules or small molecule compounds by decoding the polypeptide barcode labels associated therewith.

The method for screening polypeptide encoded nucleic acid library or polypeptide encoded small molecule compound library provided by the present invention is suitable for use with microfluidic methods and in microfluidic devices. Microfluidic devices are used to contain and transport fluid materials such as liquids, and their flow channel dimensions are at the micrometer or even nanometer level. Typical microfluidic devices generally include structural and functional units of millimeter or smaller size.

In the present invention, a single bead and/or a single or desired number of multiple cells in the library can be encapsulated in a droplet. The droplet is manipulated by a microfluidic device to achieve interaction between the candidate RNA molecules carried on the beads and a single cell or a single cell group in an enclosed micro-space, and the desired RNA molecules are screened.

FIG. 2 is an exemplary flow chart of the present invention using a microfluidic device to screen a polypeptide encoded RNA library. As shown in Figure 2, a single bead (microbead) of the library is encapsulated in a single first droplet, and the single or desired number of multiple cells is encapsulated in a single second droplet, and the cells are sorted by fluorescence-activated droplet sorting (FADS) or deterministic lateral displacement (FADS). displacement, DLD) to screen droplets containing beads or cells; treat the droplets containing beads, and make each nucleic acid molecule fall off the beads under conditions where the cleavable linker can be cleaved (such as UV irradiation) to obtain droplets containing free RNA molecules; then pair the first droplet containing a single bead and the second droplet containing a single cell, merge them to form a droplet, incubate cells in the merged droplet, observe and screen (such as by FADS) the droplets that reflect the required improved biological response, collect the screened droplets, break the droplets, separate and collect the required beads, then fall off and decode the polypeptide barcode label from the beads, and obtain the information of the corresponding RNA molecule according to the polypeptide barcode, including the sequence or the modification carried.

In one aspect of the present invention, incubating the nucleic acid molecule to be screened with multiple cells is beneficial for observing the signal generated by the influence of the nucleic acid molecule to be screened. Therefore,

before the droplet pairing, the step of obtaining droplets containing multiple cells by culturing the droplets containing a single cell or fusing multiple droplets containing a single cell can also be included.

In one aspect of the present invention, the method includes forming a droplet from a single bead or a single cell or a desired number of cells of the library, adding other reagents (such as reagents for detecting biological responses caused by the nucleic acid molecule or small molecule compound in the cell) to the solution, thereby introducing the reagents for subsequent reactions into the microenvironment (droplet) in which the cells are incubated.

In another exemplary method of the present invention using a microfluidic device to screen a polypeptide encoded RNA library, the difference from the process of FIG. 2 is that when forming a droplet, the single bead (microbead) of the library and the single or desired number of cells are encapsulated in the same droplet. Then, the droplets containing beads and cells are screened by fluorescence-activated droplet sorting (FADS) or deterministic lateral displacement (DLD); each nucleic acid molecule is detached from the beads under conditions where the cleavable linker can be cleaved (e.g., UV irradiation) to obtain droplets containing free RNA molecules, cells are incubated, droplets that reflect the desired improved biological response are observed and screened (e.g., by FADS), the screened droplets are collected, the droplets are broken, the desired beads are separated and collected, and then the polypeptide barcode label is detached from the beads and decoded, and the information of the corresponding RNA molecule, including the sequence or the modification carried, is obtained according to the polypeptide barcode.

In the following examples, the full-length mRNA library of G-CSF encoded by the polypeptide prepared in Example 1 is screened by the microfluidic sorting process as shown in FIG. 2.

The microfluidic device used includes a droplet generation unit for preparing beads containing a full-length mRNA library of G-CSF encoded by cells or polypeptides prepared in the embodiment and/or droplets containing reagents dissolved in a solution. The droplet generation unit includes an inlet for a droplet carrier oil (carrier fluid) and another inlet for a droplet aqueous phase component. It is a method known in the art to controllably encapsulate particles such as beads or cells into droplets.

FIG. 3 is a schematic diagram of an exemplary method and device for forming droplets containing a single cell. In the experiment, the flow channel height was 50μm and the width was 100μm. An aqueous solution and oil (BioRad) containing Hela cells were respectively introduced at a ratio of 7μL/min: 1μL/min. In order to perform FADS, a dye such as Calcein-AM was added to the cells.

FIG. 4A and 4B are schematic diagrams and result, respectively of exemplary screening of droplets containing a single mRNA library bead using the DLD method. The generated droplets were sorted using DLD. The flow channel is 50 μm high and 100 μm wide. The aqueous solution and oil containing the mRNA library are introduced at a ratio of 10μL/min: 13μL/min. The DLD design parameters used are: column diameter D=80μm, column spacing G=125μm, center distance of adjacent columns along the coordinate axis $\lambda$=D+G=205μm, relative offset distance of columns along the y direction $\triangle\lambda$ =8μm, and relative offset coefficient of columns along the y direction $\varepsilon$=0.03. As shown in Figure 4B, the single-package droplet containing library beads moves obliquely forward along the direction of the column array offset, and the empty-package droplet moves around the column. At the downstream screening outlet, droplets of different sizes will be located on both sides of the flow channel and enter different outlets to achieve screening of empty-package droplets.

FIG.5 is a schematic diagram of an exemplary re-injection of a first droplet containing a single bead and a second droplet containing a single cell. The droplets containing beads are irradiated with UV light, so that the linker is cleaved, the RNA molecules fall off the beads, and the droplets contain free mRNA molecules. In addition, by adjusting the period of the liquid flow into the first droplet to match the period of the liquid flow into the second droplet, the first droplet and the second droplet form a one-to-one pairing combination in the form of "ABAB" at the channel interface.

The combination of the droplet of a single bead and the droplet of a single cell merges into a droplet containing a single bead and a cell after passing through the electric field set in the microfluidic device. Electrode processing flow: The electrode is made by MEMS processing technology, which is divided into four steps: (1) SU-8 photoresist is evenly coated on the surface of the substrate by spin coating to form a photoresist coating; (2) The pre-designed electrode pattern is transferred to the substrate by photolithography; (3) By physical vapor deposition, 500 Å of titanium is first deposited on the surface of the substrate as an adhesion layer, and then 1500 Å of gold is deposited; (4) The excess photoresist and gold film are released by cleaning to obtain the pre-designed electrode. Electric field parameters: about 250V/cm, 90kHz.

In one aspect of the present invention, it is necessary to improve the activity of the free nucleic acid molecules entering the cell. In the method of the present invention, various methods known in the art for enhancing the permeability of cells to extracellular nucleic acids or small molecules can be used to promote the free nucleic acid molecules or small molecule compounds (for example, by diffusion) to enter the cell. For example, the cell membrane can be temporarily perforated by electroporation or spatial squeezing to promote the free nucleic acid molecules or small molecule compounds to enter the cell.

FIG. 6 is a schematic diagram of applying an electric field to a droplet by electroporation to cause a temporary perforation of the cell membrane to promote the free mRNA nucleic acid molecules to enter the cell. Before entering the electric field, the droplet contains cells, beads with polypeptide barcodes, and free mRNA molecules. After passing through the electric field, the free mRNA molecules enter or enter more into the cell than before passing through the electric

field. Voltage: about 6V, electric field action time, about 2.0ms.

In one aspect of the present invention, cells contained in the droplets are incubated to allow them to produce biological responses caused by the nucleic acid molecules or small molecule compounds.

FIG. 7 is an exemplary result diagram of the incubation zone of the droplets containing mRNA library beads and cells.

In this example, CISBIO's HUMAN GCSF KITS was used to detect the expression of candidate mRNA encoding GCSF in cells. GCSF Eu Cryptate Antibody and GCSF d2 Antibody were diluted 20 times, and then the two were mixed in equal volumes to a final concentration of 40pg/ml. After mixing evenly, they were input into the flow channel of the droplets containing library beads and encapsulated with the library beads to form droplets.

After passing through the electric field shown in Figure 6, the droplets containing cells, beads with polypeptide barcodes and free mRNA molecules were incubated in the incubation zone at 25 degrees for 24 hours.

Screening by FADS: The incubated droplets are sequentially passed through FACS with a detection wavelength of 665 nm, and the droplets with excitation light intensity exceeding the background are selected into the collection flow path and collected.

Purification, cutting and MS detection of selected library beads

The screened beads collected in the reaction tube are stirred loosely with a disposable dropper and poured into a 1000 mL reaction bottle (the residual resin in the reaction tube can be rinsed with ether and filtered into a 1000 mL reaction bottle). The resin has a loading capacity of 2.5mmol. After 5 minutes of ice bath, 100-150 mL of lysis solution (trifluoroacetic acid: anisole: m-cresol: ethanedithiol: water = 82.5:5:5:2.5:5) is slowly poured under magnetic stirring. Lyse under ice bath for 30 minutes, and then move to room temperature for reaction for 2.5 hours. Add 900mL of ice ether (about 10 times the volume of the lysis agent) with rapid stirring, stir at room temperature for 30 minutes, and let stand for 20 minutes.

The obtained reaction solution was poured into a G4 sand core funnel and filtered, washed twice with ether, and the filter cake was dissolved with water (25 mL×3) for polypeptide and filtered. The sample was dissolved in water and measured with MS. The results are shown in Table 1 below:

Table 1 Selected mRNA and polypeptide tag

| Number | Molecular weight | polypeptide tag | mRNA |
|---|---|---|---|
| 1 | 394.3834 | DPN | G-CSF1 |
| 2 | 351.3584 | DAF | G-CSF2 |
| 3 | 319.3570 | STL | G-CSF3 |

Example 3 Functional verification of the mRNA obtained by screening

A. Isolation and culture of PBMC

The mRNA of G-CSF 1 was selected for functional verification.

Blood from volunteers was collected and diluted to 70 mL with DPBS, and evenly divided into two 50 mL conical tubes. 10 mL of Ficoll Paque (GE Healthcare1) was gently placed under the blood layer. The test tube was centrifuged at 2000 rpm, low acceleration and low braking for 30 minutes. The tube was removed and the buffy coat PBMC layer was gently transferred to a new 50 mL conical flask and washed with DPBS. The tube was centrifuged at 1450 rpm for 10 minutes. The supernatant was aspirated and the PBMC pellet was resuspended and washed in 50 mL of DPBS. The test tube was centrifuged at 1250 rpm for 10 minutes. Repeat the washing step and resuspend the PBMC pellet in 19 mL of Optimem I (Gibco11058) and counted. The cell suspension was adjusted to a concentration of 3.0×10^6 cells/ml viable cells. Then, the cells were plated in 5 96-well tissue culture medium-treated round-bottom plates at 50 μL per well per donor. Within 30 minutes, 50 μL of the transfection mixture was added to each well. Four hours after transfection, the culture medium was supplemented with 10 μL of fetal bovine serum (Gibco10082).

B. Preparation for transfection

G-CSF1 mRNA, mRNA encoding luciferase, and TLR agonist R848 (Invivogen tlrl-r848) were diluted to 38.5 ng/μL in a final volume of 2500μL of Optimem I.

In addition, 110 μL of Lipofectamine 2000 (Invitrogen 11668-027) was diluted with 6.76 mL of Optimem I. In a 96-well plate, 9 equal portions of 135 μL of each mRNA, positive control (R-848), or negative control (Optimem I) were added to 135 μL of diluted Lipofectamine 2000. The plates containing the material to be transfected were incubated for 20 minutes. The transfection mixture was then transferred to each well of the human PBMC plate at 50 μL per well. The plates were then incubated at 37°C. At 2, 4, 8, 20, and 44 hours, each plate was removed from the incubator and the supernatant

was frozen.

After the last plate was removed, the supernatant was analyzed using a human G-CSF ELISA kit (Invitrogen KHC2032) and a human IFN-α ELISA kit (Thermo Scientific 41105-2). Each condition was performed in duplicate.

C. Protein and immune response analysis

The ability of mRNA to produce the encoded protein was assessed over time (G-CSF yield), as was the ability of mRNA to trigger innate immune recognition by measuring interferon-α production. The use of in vitro PBMC cultures is an accepted approach to measuring the immunostimulatory potential of oligonucleotides (Robbins et al., Oligonucleotides, 2009, 19: 89-102).

The results were fitted to the standard curves added to each ELISA plate using a four-parameter logistic curve. Tables 2 and 3 show the mean values of G-CSF, interferon-α (IFN-α), and tumor necrosis factor α (TNF-α) production over time from three independent PBMC donors as determined by specific ELISA.

In the G-CSF ELISA, the background signal from the Lipofectamine 2000 (LF2000) untreated condition was subtracted at each time point. The data as provided in Table 2 and Table 3 demonstrate that specific production of human G-CSF protein by human peripheral blood mononuclear cells can be significantly generated.

Table 2 Expression of selected mRNA

| Sample | G-CSF level (pg/ml) |
|---|---|
| G-CSF1 | 6122 |
| hG-CSF | 4212 |
| Luciferase | 23 |
| LF2000 | 18 |

Table 3 Activity of selected mRNA

| Sample | INF-αlevel (pg/ml) | TNF-αlevel (pg/ml) |
|---|---|---|
| G-CSF1 | 1238 | 1375 |
| hG-CSF | 1108 | 986 |
| Luciferase | 21 | 20 |
| LF2000 | 19 | 26 |

Example 4 Construction of a peptide encoded small molecule library for HDAC6 inhibitors

Fmoc-L-Asp(OAll)-Wang Resin (0.8mmol/g, 100-200 mesh, 1%DVB), label amino acids 1-20 (20 Fmoc-protected carboxylic acids) were purchased from Jier Biochemical (Shanghai) Co., Ltd., piperidine, DCC, HOBt, HATU, HOAt, and trifluoroacetic acid were all purchased from SIGMA. Other chemical reagents were purchased from Inokai Chemical Reagent Company.

Step 1 Immobilization of tag amino acid 1

Silanization of peptide reaction tube: Pour 10% trimethylsilyl chloride-toluene solution into the reaction tube overnight. Filter (the filtrate can be reused). After treatment, the reaction tube wall will not stick to the resin. Add 850 mg of resin Fmoc-L-Asp(OAll)-Wang Resin (0.8mmol/g, 100-200 mesh, 1%DVB) to the peptide synthesis tube, add 10 mL of 20% piperidine-DMF solution to the tube, stir under nitrogen for 5 min, filter, add 10 mL of 20% piperidine-DMF solution, stir under nitrogen for 20 min, filter. Add 10 mL of DCM, stir under nitrogen for 1 min, filter. Add 10 mL of DMF to each reaction tube, stir under nitrogen for 1 min, filter. Repeat the washing three times. Divide the resin into 5 parts and place them in different reactors. Add 1.5 mmol of amino acids 1 to 5 (D, S, E, M, H) respectively, add 1.5 mmol HOBT and 1.5 mmol HBTU to each tube in turn, add 10 mL DMF to each tube, shake to dissolve, and finally add 300 μL of DIPEA to each tube, shake well, and react with nitrogen for 1 h. Filter. Add 10 mL DCM, stir with nitrogen for 1 min, and filter; add 10 mL DMF to each reaction tube, stir with nitrogen for 1 min, and filter. Repeat the washing three times. Use a capillary to stick a small amount of resin (30 resin balls are enough) into a 1.5 mL centrifuge tube, add a drop of 5% ninhydrin-ethanol solution (99.9% anhydrous ethanol), and heat in a metal reaction bath at 110 °C for 3 min. If the resin balls are colorless (the resin balls are slightly red when connected to Fmoc proline) and the solution is light yellow, the reaction is complete. If the resin balls or

solution are light blue, the reaction may not be complete. Add the above-mentioned reaction reagents and repeat the process until the reaction is complete.

Step 2 Immobilization of the library molecule core

Add 10 mL of dichloromethane to each of the 5 resins prepared in step 1, blow nitrogen and stir, swell the resin for 5 minutes, filter out DCM, and add 10 mL DMF to wash once. After fully swelling with acetonitrile, add 10mM PhSiH3, Pd(PPh3)4, and 10ml DCM. After 30 minutes, filter out the solution, wash with acetonitrile, add 10 mL of dichloromethane, blow nitrogen and stir, swell the resin for 5 minutes, filter out DCM, and add 10 mL DMF to wash once. Repeat the washing three times. To the functionalized resin with AllOC (allyl ester) protection removed, the following five molecule cores as shown below were added: 1-tert-butoxycarbonyl ester-4-hydroxyproline allyl ester, 4-tert-butoxycarbonyl ester-2-hydroxypiperazine-5-carboxylic acid allyl ester, 4-tert-butoxycarbonyl ester-2-hydroxythiazine-5-carboxylic acid allyl ester, 4-tert-butoxycarbonyl ester-2-hydroxymorpholine-5-carboxylic acid allyl ester, 4-tert-butoxycarbonyl ester-2-hydroxyselenoazine-5-carboxylic acid allyl ester, 1.5 mmol each. Anhydrous acetonitrile, DCC, and HOBt were added in sequence, and the mixture was covered and reacted under nitrogen bubbling and shaking at room temperature for 24 hours.

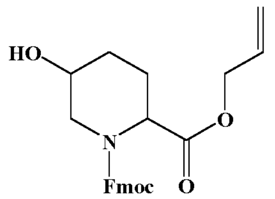
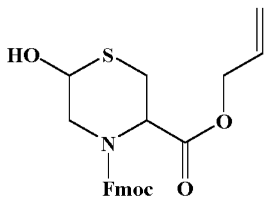
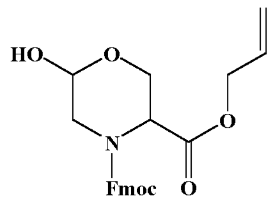

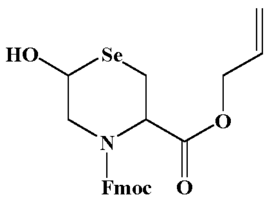
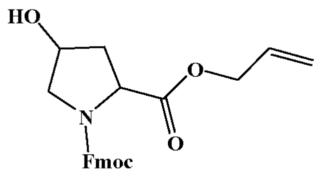

Step 3 Immobilization of amino acid tag 2

Merge the resins obtained in step 2, add 10 mL of 20% piperidine-DMF solution to the tube, stir under nitrogen for 5 min, filter, add 10 mL of 20% piperidine-DMF solution, stir under nitrogen for 20 min, filter. Add 10 mL of DCM, stir under nitrogen for 1 min, filter; add 10 mL of DMF to each reaction tube, stir under nitrogen for 1 min, filter. Repeat the washing three times. Divide the resin into 15 portions and place them in different reactors. Add 1.5 mmol of amino acids 1 to 15 (G, A, V, L, D, F, W, Y, N, K, Q, T, C, P, R) respectively, add 1.5 mmol HOBT and 1.5 mmol HBTU to each tube in turn, add 10 mL DMF to each tube, shake to dissolve, and finally add 300 μL DIPEA to each tube, shake well, activate the carboxyl group for 5 minutes, pour into reaction tubes 1 to 15, and react with nitrogen for 1 hour. Filter. Add 10 mL DCM, stir with nitrogen for 1 min, and filter; add 10 mL DMF to each reaction tube, stir with nitrogen for 1 min, and filter. Repeat the washing three times. Use a capillary to pick up a small amount of resin in a 1.5 mL centrifuge tube, add a drop of 5% ninhydrin-ethanol solution (99.9% anhydrous ethanol), and heat in a 110 °C metal reaction bath for 3 min. If the resin balls are colorless (the resin balls are slightly red when connected to Fmoc proline) and the solution is light yellow, the reaction is complete. If the resin balls or the solution are light blue, the reaction may not be complete. Add the above reaction reagents and repeat the process until the reaction is complete.

Step 4 Connection of building block 1

The following compounds are used as building block 1 of the HDAC6 small molecule library:

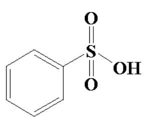
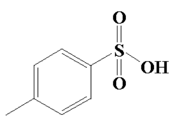
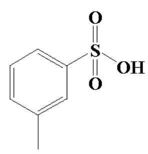

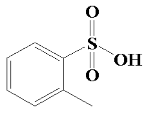 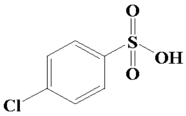 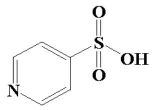 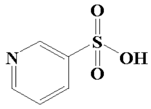

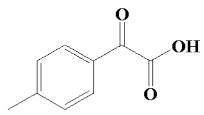 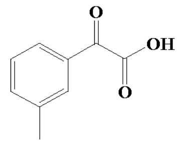 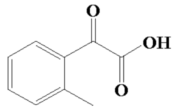 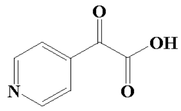

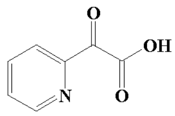 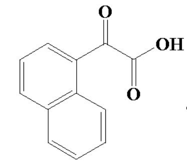 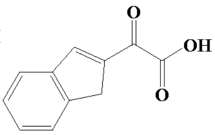 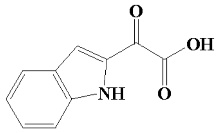 .

Add 10 mL of dichloromethane to each of the 15 portions of resin prepared in Step 3, blow nitrogen and stir, swell the resin for 5 minutes, filter out DCM, and add 10 mL of DMF to wash once. After fully swelling with acetonitrile, add 10% trifluoroacetic acid. After 30 minutes, remove the solution by suction filtration. After washing with acetonitrile, add 10 mL of dichloromethane, blow nitrogen and stir. After swelling the resin for 5 minutes, filter out DCM and add 10 mL of DMF to wash once. Repeat the washing three times. Add 1.5 mmol of 15 building blocks 1 to the functionalized resin that has been deprotected from BOC (tert-butyloxycarbonyl), add anhydrous acetonitrile, DCC, and HOBt in sequence, cover and react at room temperature with nitrogen bubbling and shaking for 24 hours.

Step 5 Immobilization of tag amino acid 3

Combine the resins obtained in step 4, add 10 mL of 20% piperidine-DMF solution to the tube, blow nitrogen and stir for 5 minutes, filter, add 10 mL of 20% piperidine-DMF solution, blow nitrogen and stir for 20 minutes, filter. Add 10 mL DCM, stir with nitrogen for 1 min, and filter; then add 10 mL DMF to each reaction tube, stir with nitrogen for 1 min, and filter. Repeat the washing three times. Divide the resin into 15 parts and place them in different reactors. Add 1.5 mmol of amino acids 1 to 15 (G, A, V, L, D, F, W, Y, N, K, Q, T, C, P, R) respectively, add 1.5 mmol HOBT and 1.5 mmol HBTU to each tube in turn, add 10 mL DMF to each tube, shake and dissolve, and finally add 300 μL of DIPEA to each tube, shake well, and react with nitrogen for 1 h. Filter. Add 10 mL DCM, stir with nitrogen for 1 min, and filter; then add 10 mL DMF to each reaction tube, stir with nitrogen for 1 min, and filter. Repeat the washing three times. Use a capillary to pick up a small amount of resin (30 resin balls are enough) in a 1.5 mL centrifuge tube, add a drop of 5% ninhydrin-ethanol solution (99.9% anhydrous ethanol), and heat in a 110 °C metal reaction bath for 3 min. If the resin balls are colorless (the resin balls are slightly red when connected to Fmoc proline) and the solution is light yellow, the reaction is complete. If the resin balls or the solution are light blue, the reaction may not be complete. Add the above reaction reagents and repeat the process until the reaction is complete.

Step 6 Connection of building block 2

The following compounds are used as building block 2 of the HDAC6 small molecule library:

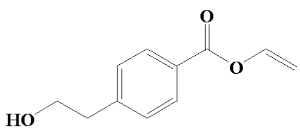 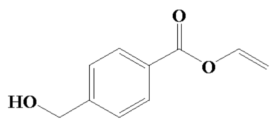 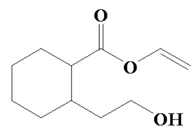

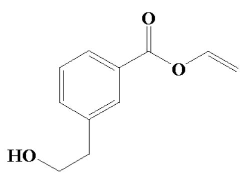 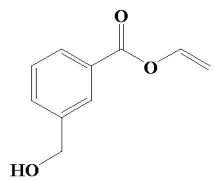 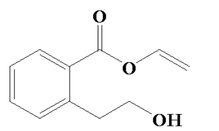

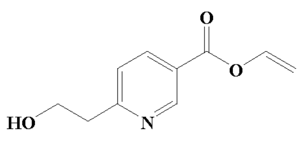 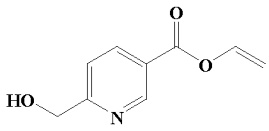 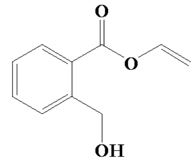

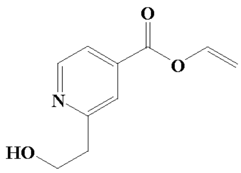 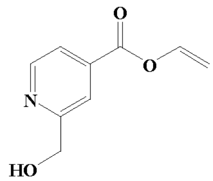 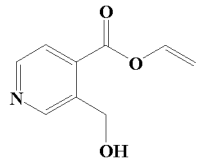

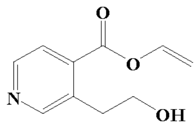 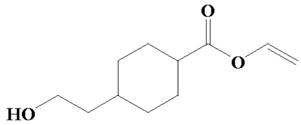 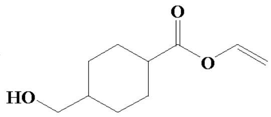 .

Add 10 mL of dichloromethane to each of the 15 portions of resin prepared in step 5, blow nitrogen and stir, swell the resin for 5 minutes, filter out DCM, and add 10 mL of DMF to wash once. After fully swelling with acetonitrile, add 10mM PhSiH3, Pd(PPh3)4, and 10ml DCM. After 30 minutes, filter out the solution, wash with acetonitrile, add 10 mL of dichloromethane, blow nitrogen and stir, swell the resin for 5 minutes, filter out DCM, and wash once with 10 mL DMF. Repeat the washing three times. Add 1.5 mmol of 15 building blocks 2 to the functionalized resin that has been deprotected from ALLOC (allyl ester group), add anhydrous acetonitrile, DCC, and HOBt in sequence, cover, and react at room temperature with nitrogen oscillation for 24 hours.

Step 7 Immobilization of tag amino acid 4

Combine the resins obtained in step 6, add 10 mL of 20% piperidine-DMF solution to the tube, blow nitrogen and stir for 5 minutes, filter, add 10 mL of 20% piperidine-DMF solution, blow nitrogen and stir for 20 minutes, and filter. Add 10 mL DCM, stir with nitrogen for 1 min, and filter; then add 10 mL DMF to each reaction tube, stir with nitrogen for 1 min, and filter. Repeat the washing three times. Divide the resin into 15 parts and place them in different reactors. Add 1.5 mmol of amino acids 1 to 15 (G, A, V, L, D, F, W, Y, N, K, Q, T, C, P, R) respectively, add 1.5 mmol HOBT and 1.5 mmol HBTU to each tube in turn, add 10 mL DMF to each tube, shake and dissolve, and finally add 300 μL of DIPEA to each tube, shake well, and react with nitrogen for 1 h. Filter. Add 10 mL DCM, stir with nitrogen for 1 min, and filter; then add 10 mL DMF to each reaction tube, stir with nitrogen for 1 min, and filter. Repeat the washing three times. Use a capillary to pick up a small amount of resin (30 resin balls are enough) in a 1.5 mL centrifuge tube, add a drop of 5% ninhydrin-ethanol solution (99.9% anhydrous ethanol), and heat in a 110 °C metal reaction bath for 3 min. If the resin balls are colorless (the resin balls are slightly red when connected to Fmoc proline) and the solution is light yellow, the reaction is complete. If the resin balls or the solution are light blue, the reaction may not be complete. Add the above reaction reagents and repeat the process until the reaction is complete.

Step 8 Connection of building block 3

The following compounds are used as building block 3 of the HDAC6 small molecule library:

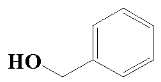 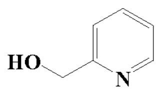 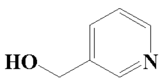 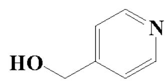 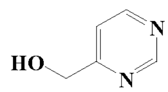

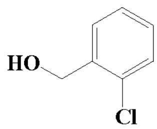 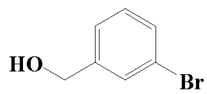 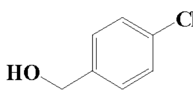 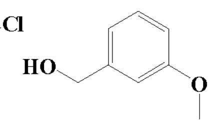 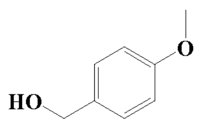

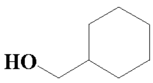 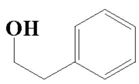 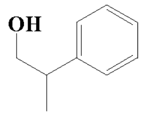 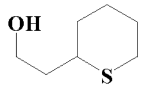 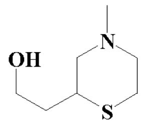 .

Add 10 mL of dichloromethane to each of the 15 portions of resin prepared in step 7, blow nitrogen and stir, swell the resin for 5 minutes, filter out DCM, and add 10 mL of DMF to wash once. After fully swelling with acetonitrile, add 10mM PhSiH3, Pd(PPh3)4, and 10ml DCM. After 30 minutes, remove the solution by suction filtration. After washing with acetonitrile, add 10 mL of dichloromethane, blow nitrogen and stir. After swelling the resin for 5 minutes, filter DCM and add 10 mL DMF to wash once. Repeat the washing three times. Add 15 building blocks 3, 1.5 mmol each, to the functionalized resin that has been deprotected by ALLOC (allyl ester group), add anhydrous acetonitrile, DCC, and HOBt in turn, cover and react at room temperature with nitrogen blowing and shaking for 24 hours. A polypeptide-encoded small molecule library with a library capacity of 5×15×15× 15=16875 was obtained.

Example 5 Screening of a polypeptide-encoded small molecule HDAC6 inhibitor library

1. Expression and purification of HDAC6 protein

Add 1 μl of recombinant pET-28a expression plasmid to competent BL21 (DE3) bacteria and place on ice for 20min; heat shock at 42°C for 90s and then spread on solid culture medium containing 30μg/ml kanamycin and culture at 37°C overnight; pick monoclonal bacteria and add them to liquid culture medium containing 30μg/ml kanamycin and 0.25mM $ZnSO_4$, and culture at 37°C with shaking until the OD600 of the bacterial solution is 0.6; add 0.5mM IPTG and induce expression at 20°C or 37°C overnight; centrifuge the bacterial solution at 4000 r/min for 10min, discard the supernatant, and resuspend the bacterial solution precipitate with PBS; ultrasonically disrupt the bacteria, collect the supernatant and precipitate by centrifugation, dissolve the precipitate with buffer solution A, and separate the supernatant and precipitate with SDS-PAGE The loading buffer solution was mixed, heated and denatured, and then SDS-PAGE electrophoresis was performed.

Repeat the above protein expression steps to induce the expression of HDAC6 protein at low temperature (20°C); after centrifugation, the bacteria were dissolved with buffer solution B, and the supernatant was collected after ultrasonic disruption; the Ni-NDA chromatography column was pre-equilibrated with 5 times the column volume of Ni-NDA binding buffer, and then the supernatant containing the target protein was loaded. Incubate for 1 hour and collect the effluent at the same time; wash the equilibrated column with 20 times the column volume of buffer C to remove endotoxin; wash the column with Ni-NDA washing buffer solution and collect the effluent at the same time; elute the target protein with Ni-NDA elution buffer solution and collect the effluent; mix the above collected effluents with SDS-PAGE loading buffer solution, heat and denature, and then perform SDS-PAGE electrophoresis. Add the purified protein to the dialysis bag and dialyze it in the protein storage buffer solution overnight. Use PEG20000 to concentrate the protein and store it in a -80°C refrigerator.

Prepare SDS-PAGE gel, load the above samples, run through the upper concentrated gel and the lower separation gel at 80 V and 120 V respectively; transfer the lower separation gel at a constant current of 300 mA, and then block the PVDF membrane in 5% skim milk powder for 1 h; dilute the His antibody at a ratio of 1: 1000, and incubate the PVDF membrane in the primary antibody dilution overnight; wash the membrane three times with TBST buffer solution, and then incubate the corresponding secondary antibody dilution solution; after washing the membrane with TBST buffer solution, use the ECL kit for development and exposure to determine the quality of protein expression.

2. Screening of compound library

200pmol of target protein was fixed on 15 μL NHS-activated agarose resin, and then the resin loaded with target protein was incubated with 100pmol DNA-encoded compound library in 100 μL PBST buffer (supplemented with salmon sperm DNA) at 4°C for 2.5 hours. After incubation, the resin was washed 7 times with 100 μL PBS to remove unbound library compounds. 20 μL H2O was added to the resin, and then the bound library compounds were eluted by heating to 95°C for 10 minutes. The resulting solution was subjected to structural analysis.

3. Purification, cutting and MS detection of selected resin

The resin in the reaction tube was stirred loosely with a disposable dropper and poured into a 1000 mL reaction bottle (the residual resin in the reaction tube can be rinsed with ether and filtered into a 1000 mL reaction bottle). The resin has a loading capacity of 2.5 mmol. After 5 minutes of ice bath, 100-150 mL of lysis solution (trifluoroacetic acid: anisole: m-cresol: ethanedithiol: water = 82.5:5:5:2.5:5) is slowly poured in under magnetic stirring. The lysis is carried out under ice bath for 30 minutes, and then moved to room temperature for reaction for 2.5 hours. Note: Do not shake the reaction bottle after adding the lysis agent to prevent the magnetic beads from hanging on the wall. Add 900 mL of ice ether (about 10 times the volume of the lysis agent) under rapid stirring, stir at room temperature for 30 minutes, and let it stand for 20 minutes. Pour the reaction solution in the previous step into a G4 sand core funnel and filter it. Wash it with ether twice, dissolve the peptide in water (25 mL×3) and filter it. Dissolve the sample in water and measure MS.

The results are shown in Table 4 below.

Table 4 Screened small molecule compounds and peptide tags

| Number | MW of peptide tag | Peptide tag | Structure of compound |
|---|---|---|---|
| 1 | 594.68 | ERYQ | 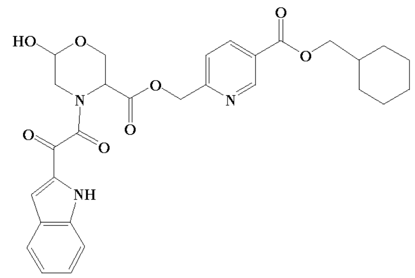 |
| 2 | 469.53 | HGRA | 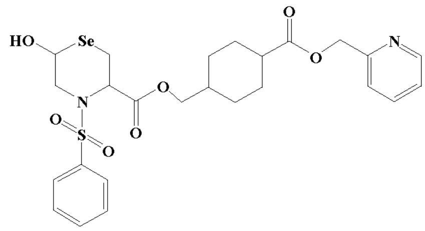 |
| 3 | 416.52 | DAVL | 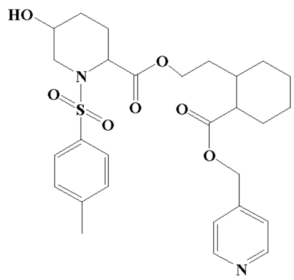 |
| 4 | 480.61 | DLCL | 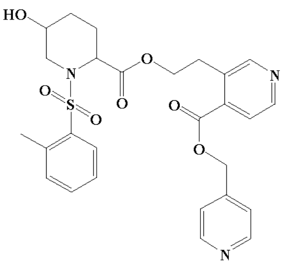 |
| 5 | 468.51 | SFTD | 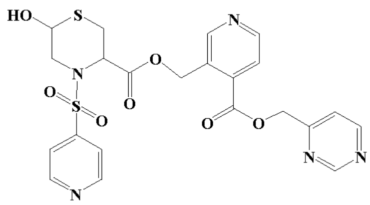 |

(continued)

| Number | MW of peptide tag | Peptide tag | Structure of compound |
|---|---|---|---|
| 6 | 582.66 | SYQW | 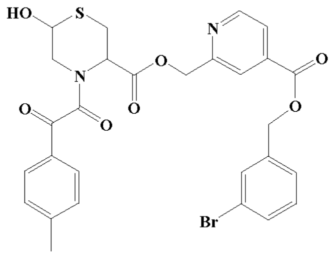 |
| 7 | 494.59 | ETVF | 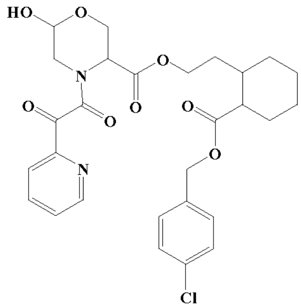 |
| 8 | 552.63 | EKNY | 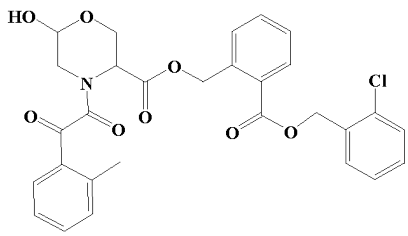 |
| 9 | 514.58 | DAYF | 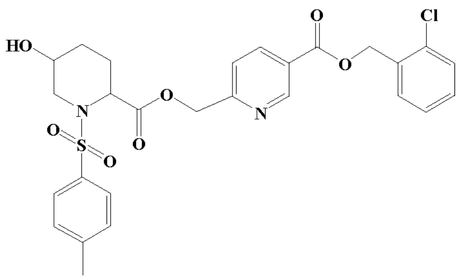 |
| 10 | 464.56 | DALF | 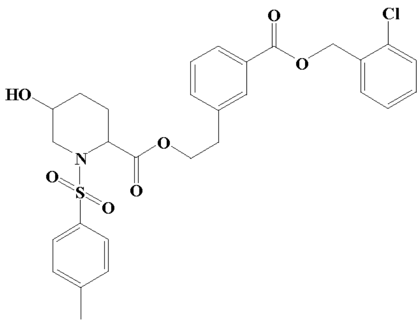 |

(continued)

| Number | MW of peptide tag | Peptide tag | Structure of compound |
|---|---|---|---|
| 11 | 574.70 | DRVW | 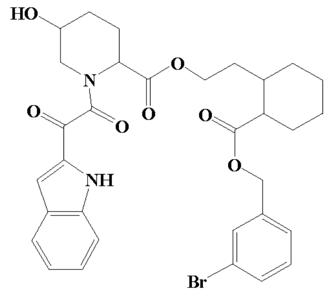 |
| 12 | 597.73 | HQWK | 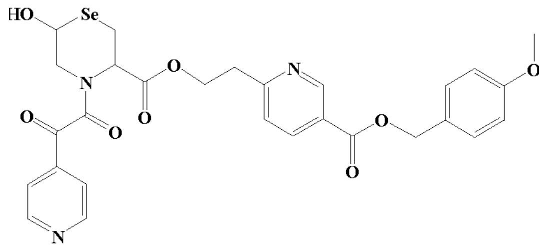 |
| 13 | 482.54 | DTFT | 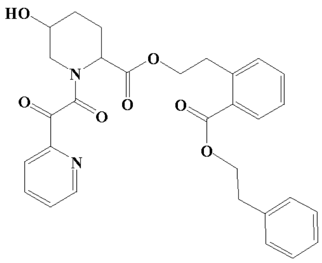 |
| 14 | 514.66 | HCKQ | 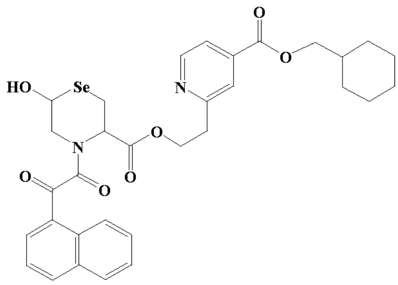 |
| 15 | 527.61 | DCYQ | 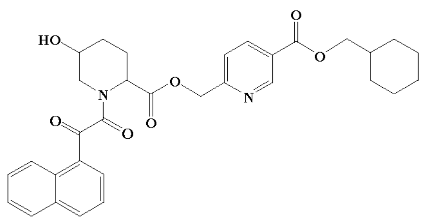 |
| 16 | 462.53 | EPDC | 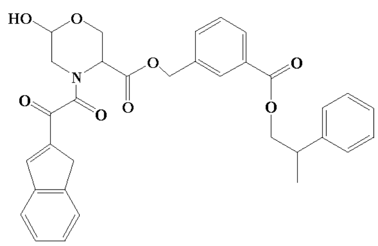 |

(continued)

| Number | MW of peptide tag | Peptide tag | Structure of compound |
|---|---|---|---|
| 17 | 457.53 | DPLN | 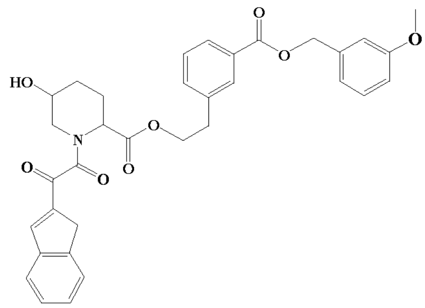 |
| 18 | 447.57 | STKL | 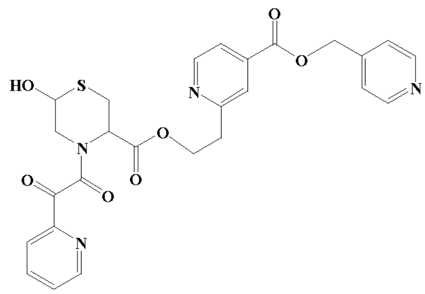 |
| 19 | 431.54 | SRVA | 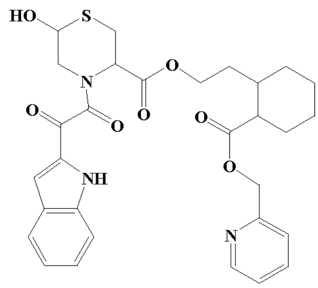 |
| 20 | 509.61 | DTKF | 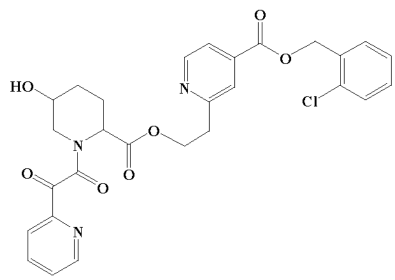 |

Example 6 Functional verification of selected compounds

The compounds to be tested were balanced to room temperature, and the compounds were diluted 4 times with DMSO in a 384-well plate, with a starting working concentration of 250nM and a total of 7 concentration gradients. 250nl of the diluted drug solution was transferred to the 384-well reaction plate using Echo 550, where 250nl of DMSO was transferred to the control wells Max and Min wells; the HDAC6 enzyme stock solution was diluted to 1.67× with 1× enzyme reaction system buffer solution; the reaction substrate was diluted to 2.5× with 1× enzyme reaction system buffer solution; 15μl of the above diluted enzyme working solution was added to each reaction well, and 15μl of 1× enzyme reaction system buffer solution was added to the Min well. Incubate at room temperature for 15 min; add 10μl of the above-diluted enzyme reaction substrate to each reaction well and incubate at room temperature for 4 hours to allow the reaction to proceed fully; add 3.5×Trypsin reaction stop solution to each reaction well and incubate at 37°C for 90 min to terminate the reaction; read the fluorescence signal at the end of the reaction on the microplate reader.

The calculation formula for the inhibition rate of the compound on HDAC6 is as follows:

$$\% \, Inhibition = \frac{Mean(Max) - Sample\ Signal}{Mean(max) - Mean(Min)} \times 100\%$$

Where Mean (Max) is the mean of the detection values of the sample wells of each Max well (only DMSO and enzyme); Mean (Max) represents the average value of the detection values of each Min well (without enzyme); Sample Signal is the mean of the detection values of each compound-treated well. The dose-effect curve was fitted using GraphPad Prism software, and the IC50 of the compound on enzyme inhibition was calculated (as shown in Table 5).

Table 5 Inhibitory activity of preferred compounds on HDAC6

| Number | IC50(nM) | Bottom | Top | Hill Slope |
|---|---|---|---|---|
| 1 | 0.63 | -8 | 96 | 1.2 |
| 2 | 0.85 | -6 | 99 | 1.1 |
| 3 | 2.6 | -2 | 100 | 1.5 |
| 4 | 13.28 | 0 | 91.4 | 1.06 |
| 5 | 4 | -2 | 99 | 1 |
| 6 | 0.75 | -4 | 100 | 1.1 |
| 7 | 0.85 | -8 | 102 | 0.9 |
| 8 | 2.9 | -3 | 101 | 1.5 |
| 9 | 19.41 | -2.897 | 96.38 | 0. 8205 |
| 10 | 1.5 | 3 | 98 | 0.8 |
| 11 | 0.23 | -6 | 100 | 1 |
| 12 | 0.41 | -2 | 101 | 1.1 |
| 13 | 2.2 | -2 | 98 | 1.6 |
| 14 | 132 | 1 | 100 | 0.8 |
| 15 | 5.986 | -5.009 | 106 | 0.8638 |
| 16 | 3.9 | 3 | 99 | 1 |
| 17 | 0.58 | 3 | 102 | 1.1 |
| 18 | 0.92 | 0 | 102 | 1 |
| 19 | 2.6 | -3 | 96 | 1.6 |
| 20 | 53.78 | 5 | 99.3 | 0.83 |

Although the present invention has been described in detail with reference to the above exemplary embodiments, one skilled in the art may make various modifications of the embodiments, and substitute the parts herein with equivalence without departing from the scope of the present invention. The present invention is not limited to the specific embodiments disclosed herein, and shall include all technical solutions falling within the scope of the claims of the present invention.

## Claims

1. A polypeptide-encoded nucleic acid or small molecule compound library, the library comprising a plurality of nucleic acid molecules or small molecule compounds, wherein each of the nucleic acid molecules or small molecule compounds has a corresponding polypeptide barcode tag.

2. The library according to claim 1, comprising a plurality of beads, each bead comprising a polypeptide barcode tag connected to the bead and a nucleic acid molecule or small molecule compound corresponding to the polypeptide barcode tag, and optionally, the nucleic acid molecule or small molecule compound being releasably connected to the bead.

3. The library according to claim 1 or 2, wherein the polypeptide barcode tag is formed by n amino acid residue building blocks sequentially connected to each other, wherein n is an integer of 3-10, preferably 4-8, and for example, n is 4 or 5 or 6; and
the nucleic acid molecule is formed by m nucleic acid units sequentially connected to each other, or the small molecule compound is composed of m compound building blocks, wherein m is the same as the number n of amino acid residue building blocks in the polypeptide barcode tag associated with the corresponding bead.

4. The library according to claim 3, wherein each of the nucleic acid units and each of the amino acid residue building units are sequentially and alternately assembled on the bead, or each of the compound building units and each of the amino acid residue building units are sequentially and alternately assembled on the bead.

5. The library according to claim 2, wherein the bead is a hydrogel particle, such as a gel particle of polyacrylamide, agarose, alginate and other gel particles.

6. The library according to claim 1 or 2, wherein each of the amino acid residue units in the polypeptide barcode tag comprises one or more (e.g., 2-8) amino acid residues, and for example, the amino acid residue unit comprises 1 or 2 amino acid residues.

7. The library according to claim 1, wherein the library is an mRNA or RNAi library.

8. The library according to claim 7, wherein the mRNA is formed by m mRNA units sequentially connected to each other, wherein m is the same as the number n of amino acid residue units in the polypeptide barcode tag associated with the corresponding bead.

9. The library according to claim 8, wherein the mRNA unit comprises an mRNA coding sequence or a fragment thereof, a flanking region of the mRNA (such as a 5’ untranslated region, i.e., 5’UTR, or a 3’ untranslated region, i.e., 3’UTR) and/or a terminal region (such as a 5’ cap, a poly A tail).

10. The library according to claim 7, wherein the mRNA is an mRNA encoding a target protein or a variant thereof, such as an mRNA encoding an antibody or a fragment thereof, a vaccine or a therapeutic protein.

11. The library according to claim 2, wherein the nucleic acid molecules or small molecule compounds in the library have a cleavable linker releasably connected to the bead, the cleavable linker being one selected from a group consisting of: an enzymatically cleavable linker; a nucleophile/base sensitive linker; a reduction sensitive linker; a photocleavable linker; an electrophile/acid sensitive linker; a metal-assisted cleavage sensitive linker; an oxidation sensitive linker; and a combination of two or more of the foregoing, such as an ultraviolet cleavage linker.

12. A method for screening a polypeptide-encoded nucleic acid or small molecule compound library, wherein each of the nucleic acid molecules or small molecule compounds has a corresponding polypeptide barcode tag,
the method comprising identifying a corresponding nucleic acid molecule or small molecule compound by decoding the polypeptide barcode tag, wherein the library of nucleic acid molecules or small molecule compounds is contact with a biological target, the biological target comprising, for example, cells, and biological macromolecules such as proteins, nucleic acids and polysaccharides.

13. The method according to claim 12, further comprising contacting the library with a biological target under conditions suitable for exposing the nucleic acid molecules or small molecule compounds of the library to the biological target, optionally comprising removing library members that do not bind to the target, and analyzing the tags associated with the small molecule nucleic acid molecules or small molecule compounds bound to the target.

14. The method according to claim 12, wherein the library comprises a plurality of beads, each bead comprising a polypeptide barcode tag connected to the bead and a nucleic acid molecule or a small molecule compound corresponding to the polypeptide barcode tag, and optionally, the nucleic acid molecule or the small molecule compound being releasably connected to the bead.

15. The method according to claim 14, wherein the method comprises steps of:

(a) providing the library comprising a plurality of beads having different polypeptide barcode tags and corresponding nucleic acid molecules or small molecule compounds;

(b) releasing the nucleic acid molecules or small molecule compounds of each bead from the bead to produce a plurality of free nucleic acid molecules or small molecule compounds;
(c) contacting the nucleic acid molecules or small molecule compounds of each bead with a single or multiple biological targets (e.g., cells) to screen the nucleic acid molecules or small molecule compounds, in order to produce screened nucleic acid molecules or small molecule compounds; and
(d) identifying the screened nucleic acid molecules or small molecule compounds by decoding the polypeptide barcode tags associated therewith,
wherein, preferably, the beads are hydrogel particles, such as polyacrylamide, agarose, alginate and other gel particles,
optionally, step (d) comprises a step of removing the polypeptide barcode tags from the beads,
and, optionally, step (b) comprises releasing each nucleic acid molecule from the beads under conditions where the cleavable linker is cleavable, in order to produce a plurality of free nucleic acid molecules.

**16.** The method according to claim 15, wherein step (a) comprises encapsulating different beads of the library in a plurality of first discrete entities, wherein preferably, a single one of the plurality of first discrete entities statistically contains one bead,

wherein step (c) comprises encapsulating the biological target (e.g., cell) in a plurality of second discrete entities, wherein preferably, a single one of the plurality of second discrete entities statistically contains one cell or a desired number of cells, and
preferably, the discrete entities are droplets.

**17.** The method according to claim 16, comprising sorting a first discrete entity containing the beads or a second discrete entity containing the biological target (e.g., cell) from the discrete entities such as droplets, wherein the sorting is performed, for example, by fluorescence-activated droplet sorting (FADS) or deterministic lateral displacement (DLD).

**18.** The method according to claim 16, wherein a single bead of the library is encapsulated in a single first droplet, a single or a desired number of cells are encapsulated in a single second droplet, and then the first droplet containing the bead and the second droplet containing the cell are merged to form a droplet.

**19.** The method according to claim 15, wherein step (d) comprises one or more steps of:

collecting the screened droplets;
breaking the droplets;
separating and collecting the beads; and
removing the polypeptide barcode label from the beads.

**20.** A polypeptide encoded discrete entity library of nucleic acids or small molecule compounds, comprising beads of a polypeptide-encoded nucleic acid or small molecule compound library as defined in any one of claims 1-11, wherein the beads are encapsulated in a single discrete entity, and preferably, the discrete entity is a droplet.

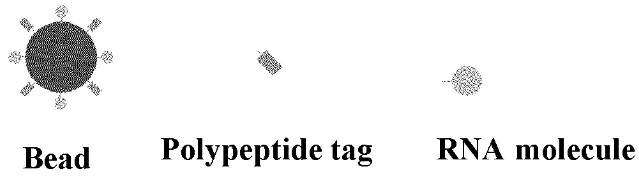


FIG. 1

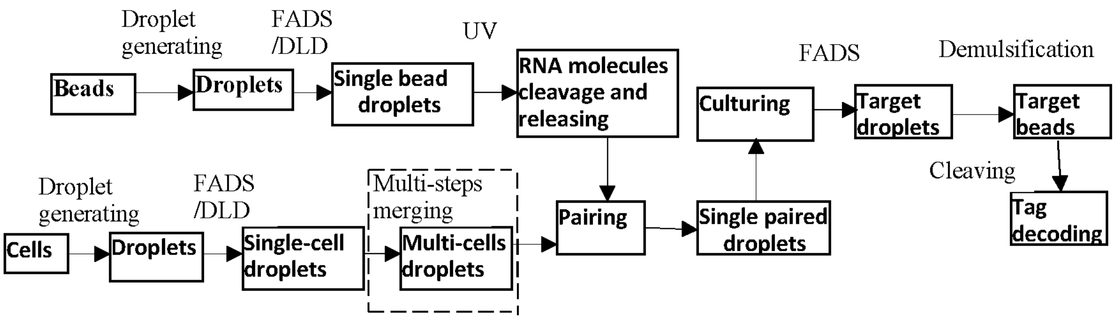


FIG. 2

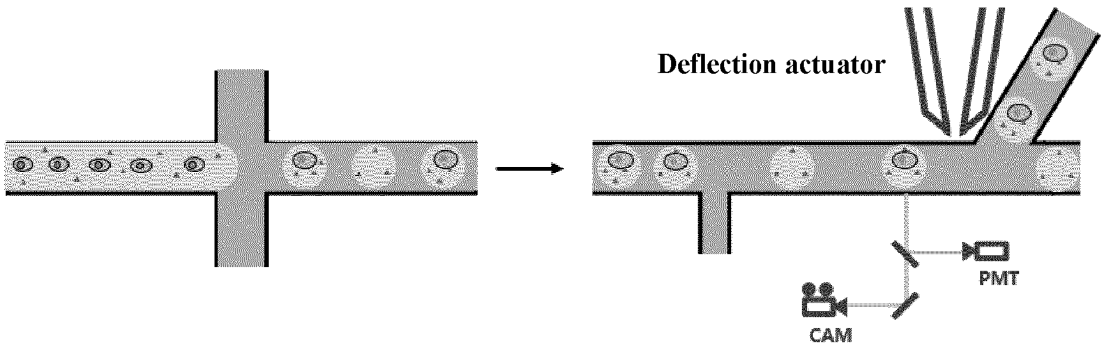


FIG. 3

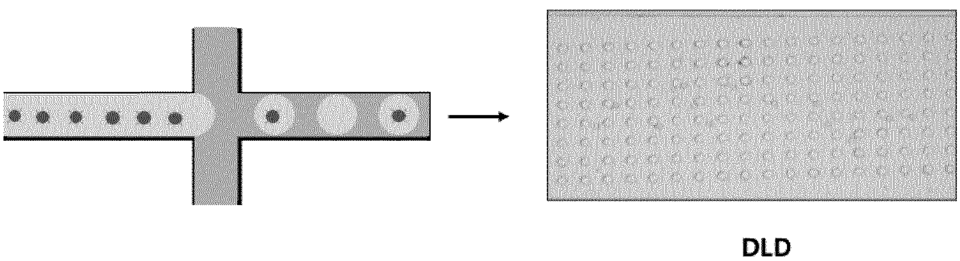


FIG. 4A

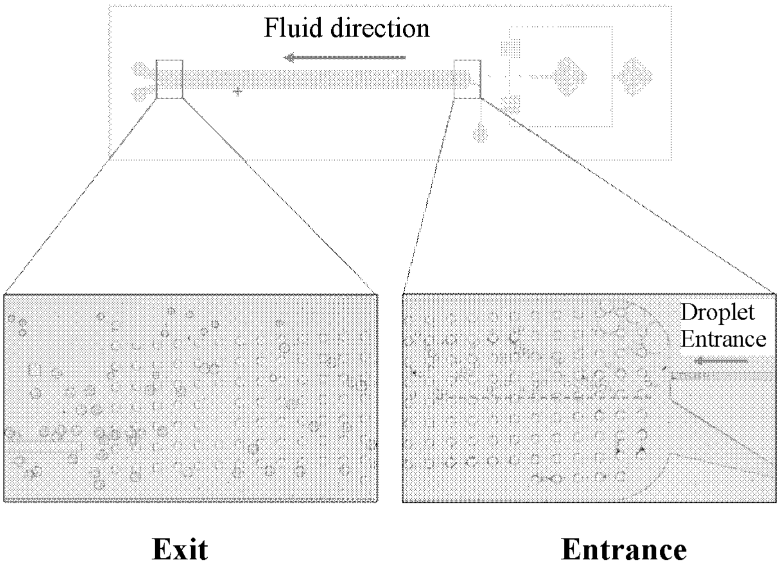


FIG. 4B

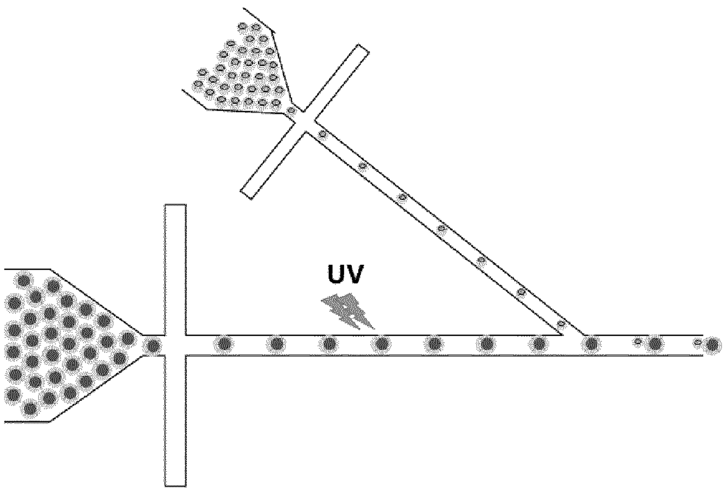


FIG. 5

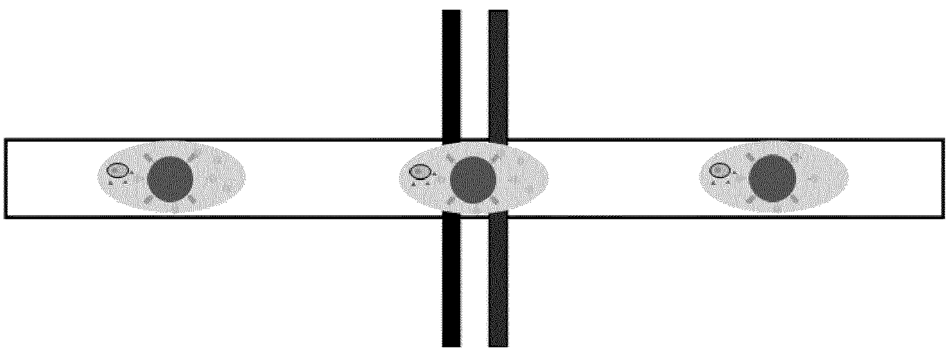

FIG. 6

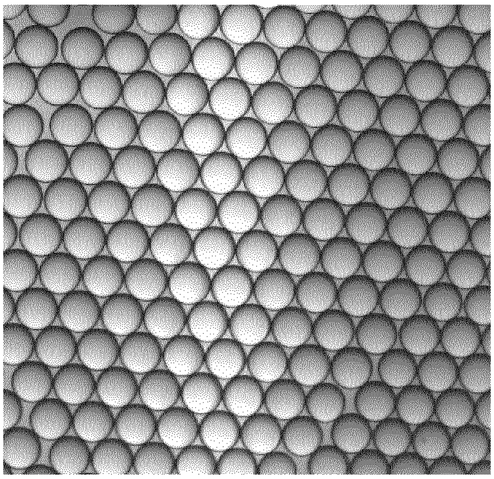

FIG. 7

1 agcccggagc ctgcagccca gccccaccca gacccatggc tggacctgcc acccagagcc
61 ccatgaagct gatggccctg cagctgctgc tgtggcacag tgcactctgg acagtgcagg
121 aagccacccc cctgggccct gccagctccc tgccccagag cttcctgctc aagtgcttag
181 agcaagtgag gaagatccag ggcgatggcg cagcgctcca ggagaagctg gtgagtgagg
241 caggctgctt gagccaactc catagcggcc ttttcctcta ccaggggctc ctgcaggccc
301 tggaagggat ctcccccgag ttgggtccca ccttggacac actgcagctg gacgtcgccg
361 actttgccac caccatctgg cagcagatgg aagaactggg aatggcccct gccctgcagc
421 ccacccaggg tgccatgccg gccttcgcct ctgctttcca gcgccgggca ggaggggtcc
481 tggttgcctc ccatctgcag agcttcctgg aggtgtcgta ccgcgttcta cgccaccttg
541 cccagccctg agccaagccc tccccatccc atgtatttat ctctatttaa tatttatgtc
601 tatttaagcc tcatatttaa agacagggaa gagcagaacg gagccccagg cctctgtgtc
661 cttccctgca tttctgagtt tcattctcct gcctgtagca gtgagaaaaa gctcctgtcc
721 tcccatcccc tggactggga ggtagatagg taaataccaa gtatttatta ctatgactgc
781 tccccagccc tggctctgca atgggcactg ggatgagccg ctgtgagccc ctggtcctga
841 gggtccccac ctgggaccct tgagagtatc aggtctccca cgtgggagac aagaaatccc
901 tgtttaatat ttaaacagca gtgttcccca tctgggtcct tgcacccctc actctggcct
961 cagccgactg cacagcggcc cctgcatccc cttggctgtg aggcccctgg acaagcagag
1021 gtggccagag ctgggaggca tggccctggg gtcccacgaa tttgctgggg aatctcgttt
1081 ttcttcttaa gacttttggg acatggtttg actcccgaac atcaccgacg cgtctcctgt
1141 ttttctgggt ggcctcggga cacctgccct gcccccacga gggtcaggac tgtgactctt
1201 tttagggcca ggcaggtgcc tggacatttg ccttgctgga cggggactgg ggatgtggga
1261 gggagcagac aggaggaatc atgtcaggcc tgtgtgtgaa aggaagctcc actgtcaccc
1321 tccacctctt caccccccac tcaccagtgt cccctccact gtcacattgt aactgaactt
1381 caggataata aagtgtttgc ctcca

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2016080341 W


### Non-patent literature cited in the description


- **BARET et al.** *Lap Chip*, 2009, vol. 9, 1850-1858
- **ROBBINS et al.** *Oligonucleotides*, 2009, vol. 19, 89-102